# EUROPEAN PATENT SPECIFICATION

(11) **EP 1 750 788 B1**
(45) Date of publication and mention of the grant of the patent: **08.01.2014**
(21) Application number: 04754583.5
(22) Date of filing: 03.06.2004
(51) Int. Cl.: A61M 11/04, A61M 15/00

(54) **MULTIPLE DOSE CONDENSATION AEROSOL DEVICES AND METHODS OF FORMING CONDENSATION AEROSOLS**
MULTIDOSIS-KONDENSATIONSAEROSOLVORRICHTUNGEN UND VERFAHREN ZUR HERSTELLUNG VON KONDENSATIONSAEROSOLEN
DISPOSITIF AEROSOL A CONDENSATION MULTIDOSE ET PROCEDES DE FORMATION D'AEROSOLS A CONDENSATION

(43) Date of publication of application: 14.02.2007
(73) Proprietor: Alexza Pharmaceuticals, Inc., Mountain View, CA 94043 (US)
(72) Inventor: CROSS, Steven, D., Alamo, CA 94507 (US); HERBETTE, Matthieu, Sunnyvale, CA 94086 (US); KELLY, Andrew, J., G., Palo Alto, CA 94301 (US); MYERS, Daniel, J., Mountain View, CA 94041 (US); SHEN, William, W., Stanford, CA 94305 (US); TIMMONS, Ryan, D., Mountain View, CA 94040 (US); TOM, Curtis, San Mateo, CA 94402 (US); VIRGILI, Justin, M., Berkeley, CA 94703 (US); WENSLEY, Martin, J., Los Gatos, CA 95032 (US)
(74) Representative: Clegg, Richard Ian
(86) International application number: PCT/US2004/018015
(87) International publication number: WO 2005/120614

(56) References cited:
- WO-A1-02/098496
- WO-A1-03/049535
- WO-A2-02/051469
- WO-A2-03/037412

## Description

This disclosure relates to devices capable of entraining a substance into an airflow, to articles and methods employing such devices, and in particular to articles and methods of producing multiple doses of a condensation aerosol of a drug having high purity, high yield, characterized by a particle size distribution suitable for inhalation delivery, and which can be administered to a user during a single inhalation.

Pulmonary delivery is known as an effective way to administer physiologically active compounds to a patient for the treatment of diseases and disorders. Devices developed for pulmonary delivery generate an aerosol of a physiologically active compound that is inhaled by a patient where the compound can be used to treat conditions in a patient's respiratory tract and/or enter the patient's systemic circulation. Devices for generating aerosols of physiologically active compounds include nebulizers, pressurized metered-dose inhalers, and the dry powder inhalers. Nebulizers are based on atomization of liquid drug solutions, while pressurized metered-dose inhalers and dry powder inhalers are based on suspension and dispersion of dry powder in an airflow and/or propellant.

Aerosols for inhalation of physiologically active compounds can also be formed by vaporizing a substance to produce a condensation aerosol comprising the active compounds in an airflow. A condensation aerosol is formed when a gas phase substance formed from vaporization condenses or reacts to form particulates (also called particles herein) in the air or a gas. Examples of devices and methods employing vaporization methods to produce condensation aerosols are disclosed in U.S. Patent Nos. 6,682,716; 6,737,042; 6,716,415; 6,716,416; 6,740,307; 6,740,308; 6,737,043; 6,740,309; and 6,716,417.
WO02/098496 relates to the inhalation delivery of aerosols containing small particles and discloses a device that forms drug containing aerosols for use in inhalation therapy.

It can be desirable that an inhalation device be capable of delivering multiple doses of a physiologically active compound and that each dose comprising the active compound be administered to a patient during a single inhalation. A dose refers to the amount of a substance released during one activation of an inhalation device. A dose can comprise, for example, a therapeutically effective amount of a physiologically active compound. Furthermore, treatment regimens can require that each of the multiple doses delivered to a patient comprise a controlled amount of a physiologically active compound, and that the active compound administered exhibit high purity and be free of byproducts, e.g., excipients. Optimal delivery of a dose to a patient's respiratory tract, and in particular to a patient's lungs, can also be facilitated by the aerosol having a mass median aerodynamic diameter of less than about 4 µm. Furthermore, practical considerations make it desirable that a substantial amount of each dose contained in the device, form an aerosol, be emitted from the device, and be inhaled by the patient.

When a condensation aerosol is formed in an airflow, a certain portion of the aerosol can deposit on downstream physical features such as the side walls of the airway defining the airflow, the mouthpiece of the device, or other structures and thereby reduce the amount of active compound emitted by the device and available for administration. In multiple dose devices, packaging the multiple doses within a common airway can be attractive for producing low cost and compact products. However, in multiple dose devices, where the multiple doses are disposed on surfaces within an airflow, a certain amount of an aerosol particles formed by vaporizing an upstream dose, can deposit onto downstream surfaces comprising unvaporized compound. Not only can the deposition on unvaporized doses reduce the amount of active compound emitted from the device, but in addition, the deposition can change the amount of active compound forming subsequent doses. Thus, particularly where a device includes a large number of multiple doses, the latter doses can comprise a variable and uncontrolled amount of an active compound.

For many treatment regimens, the ability to deliver a dose comprising a precise, consistent, and reproducible amount of a physiologically active compound can impact the therapeutic efficacy of the treatment regimens, and in some cases, such a capability can also enable new therapies. Thus, there is a need for inhalation devices and methods of producing a condensation aerosol that can repeatedly deliver precise, reproducible and/or controlled amounts of a physiologically active substance.

The invention provides devices for entraining a substance within an airflow comprising an airway with an inlet, and an outlet; a plurality of supports disposed within the airway; the substance disposed on the at least one support; and a mechanism configured to vaporise the substance from the at least one support upon the application of mechanical, accoustic, radiation, radio frequency, optical and/or thermal energy, wherein an airflow passing from the inlet to the outlet is directed to the at least one support such that the substance is entrained in the airflow when released from the support to form a condensation aerosol in the airflow.

Certain embodiments include electrically resistive heating elements comprising a metal foil for vaporizing a substance disposed thereon to produce a condensation aerosol comprising the substance.

Certain embodiments include devices for delivering a condensation aerosol to a subject comprising a dispensing unit and a separable cartridge. In certain embodiments, the dispensing unit comprises a first housing comprising a receptacle for a separable cartridge; a controller for controlling vaporization of the substance; and a power source. In certain embodiments, the separable cartridge comprises a second housing; an airway contained within the housing having an inlet, and an outlet; a mouthpiece coupled to the outlet; an air bypass hole coupled to the outlet; at least one electrically resistive heating element disposed within the airway; a substance disposed on the at least one heating element; and an actuation mechanism configured to transfer energy from the power source to the at least one heating element; wherein an airflow from the inlet to the outlet of the airway causes the substance to vaporize and condense in the airflow to form a condensation aerosol.

Certain embodiments include methods of entraining a vaporized substance or aerosol particles into an airflow, methods of producing a condensation aerosol, and methods of administering a substance to a subject using the devices disclosed herein. For purposes herein, "entrain" or "entraining" means to direct, lift, draw in or along, inject, transport, carry, or suspend a vaporized substance or aerosol particle into an airflow.

Other embodiments will be apparent to those skilled in the art from consideration and practice of the invention disclosed herein. It is intended that the specification and examples be considered as exemplary only.

It is to be understood that both the foregoing general description and the following detailed description are exemplary and explanatory only and are not restrictive of certain embodiments, as claimed.

### Description of the Drawings

Fig. 1A is a schematic illustration showing deposition of a substance on downstream surfaces.
Fig. 1B is a schematic illustration showing the use of an airflow through a plurality of holes to entrain a substance into an airflow and thereby minimize deposition of the substance on downstream surfaces according to certain embodiments.
Figs. 2A-2F are schematic illustrations showing examples of airflow routing in a device for entraining a condensation aerosol particle into an airflow according to certain embodiments.
Fig. 3 is an isometric diagram of a separable cartridge for an electric multi-dose condensation aerosol delivery device.
Fig. 4 shows the airflow rate in the airway for different total airflow rates for a cartridge.
Fig. 5 is a schematic cross-sectional illustration of a separable cartridge for an electric multi-dose condensation aerosol delivery device showing the routing of the airflow according to certain embodiments.
Figs. 6A and 6B show views of a structure separating the first airway and the second airway according to certain embodiments.
Fig. 7 is a isometric view of an electric multi-dose condensation aerosol delivery device.
Fig. 8 is a cut-away isometric view of a portion of an electric multi-dose condensation aerosol delivery device.
Fig. 9 is an isometric view of a dispensing unit for an electric multi-dose condensation aerosol delivery device.
Fig. 10 is a schematic illustration showing a view of an arched metal foil according to certain embodiments.
Fig. 11 shows an example of the distortion of a flat metal foil, and an arched metal foil before and during resistive heating.
Fig. 12 is a partial cross-sectional view of a separable cartridge including air routing according to certain embodiments.
Fig. 13 is a block diagram of an embodiment the electrical functions for an electric multi-dose condensation aerosol delivery device.
Fig. 14 shows the particle size distribution of a condensation aerosol comprising a substance emitted from an electric multi-dose condensation aerosol delivery device according to certain embodiments.
Fig. 15 shows the reproducibility of the amount and purity of doses of fentanyl emitted from a new, an opened, and a partially-used electric multi-dose condensation aerosol delivery device according to certain embodiments.
Fig. 16 shows a temperature profile of a metal foil in an airflow according to certain embodiments.
Figs. 17A and 17B show the temperature uniformity of a metal foil in an airflow with fentanyl as the substance according to certain embodiments.
Fig. 18 shows the amount of substance deposited on downstream heating elements from vaporized substances from preceding heating elements for different airflow velocities with iittie or no airflow directed upward from underneath the heating elements.
Fig. 19 shows the amount of substance deposited on downstream heating elements from vaporized doses with a percentage of the total airflow directed upward from underneath the heating elements, where the airflow distribution was controlled by a layer of foam between the first and second airways.
Figs. 20A and 20B show a relationship between the temperature of a metal foil and the purity and amount of the dose emitted from an electric multi-dose condensation aerosol delivery device according to certain embodiments.

### Description of Various Embodiments

Unless otherwise indicated, all numbers expressing quantities and conditions, and so forth used in the specification and claims are to be understood as being modified in all instances by the term "about."

In this application, the use of the singular includes the plural unless specifically stated otherwise. In this application, the use of "or" means "and/or" unless stated otherwise. Furthermore, the use of the term "including," as well as other forms, such as "includes" and "included," is not limiting.

Condensation aerosols can be formed when a gaseous substance condenses or reacts to form particulates in air or a gas. A gaseous substance can be produced when a solid or liquid substance is thermally sublimed or vaporized. Vaporization refers to a phase transition in which a substance changes from a solid or liquid state into a gaseous state. Sublimation refers to a phase transition in which a substance passes directly from a solid state to a gaseous state.

Upon entering an airflow, a gaseous substance can cool and, at least in part depending on the temperature of the airflow, can condense to form an aerosol particle. Condensation aerosol particles not sufficiently entrained within the airflow have a greater probability of falling out of the airflow to deposit on a downstream surface.

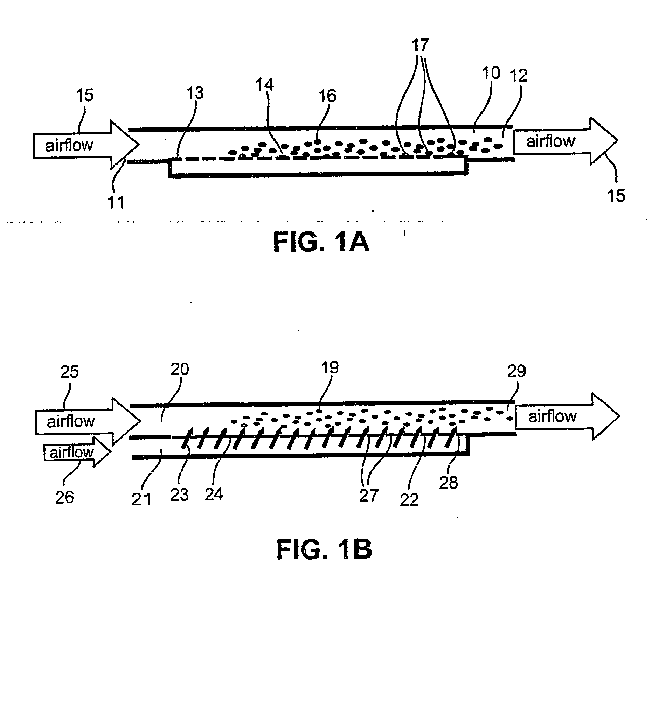

Inefficient entrainment of particulates within an airflow and subsequent deposition of the particulates on downstream surfaces is shown in **Fig. 1A. Fig. 1A** shows an airway **10** having an inlet **11** and an outlet **12.** A plurality of supports **13** are located on one side of airway **10.** Plurality of supports **13** include support **14** and downstream supports **17.** A substance can be disposed, for example, on support **14,** and an airflow **15** established in airway **10** such that plurality of supports **13** including support **14** are disposed in airflow **15.** When the substance disposed on support **14** is released from support **14** by, for example, vaporization, the substance can form condensation aerosol particles **16** in airflow **15.** As shown, when the aerosol particles are not fully entrained within airflow **15,** condensation aerosol particles **16** so formed can deposit on downstream supports **17.**

A schematic illustration of a device for entraining a particulate, and in particular an aerosol-forming gas phase substance, within an airflow is shown in **Fig. 1B**. **Fig. 1B** shows a first airway **20** and a second airway **21** separated by a structure **22.** Structure **22** comprises a plurality of holes fluidly connecting first airway **20** and second airway **21.** A plurality of supports **28** including upstream support **24,** and downstream supports **27** are disposed on the surface of structure **22** within first airway **20.** As in **Fig. 1A****,** a substance can be disposed, for example, on upstream support **24.** A first airflow **25** can be established in first airway **20,** and a second airflow **26** can be established in second airway **21** such that second airflow **26** passes from second airway **21** to first airway **20** through the plurality of holes as indicated by the upward pointing arrows **23.** Upon passing through the plurality of hole, second airflow **26** can provide a flow of air directed toward plurality of supports **28,** including upstream support **24** and directed toward airflow **25.** The flow of air **23** directed toward airflow **25** can act to lift a substance vaporized from upstream support **24** to form condensation aerosol particles **19** comprising the substance, and entrain the condensation particles within first airflow **25.** Entrainment of condensation particles **19** within first airflow **25** will reduce the likelihood that the condensation particles **19** will become deposited on the downstream surfaces **27.** As shown in **Fig 1B****,** by entraining the condensation particles near the center of first airflow **25,** more of the condensation particles can be emitted as an aerosol from the outlet **29** of the device and be available, for example, for administration to a subject by inhalation.

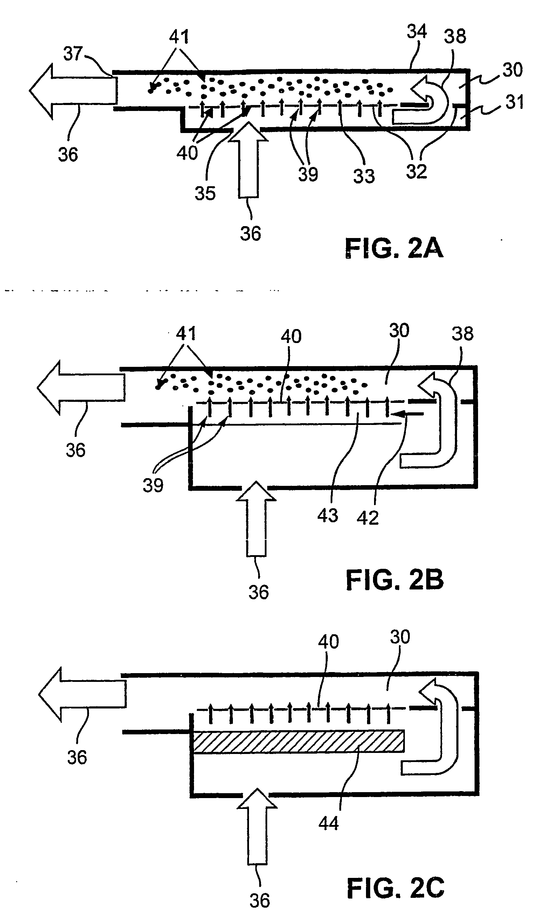

Another embodiment of a device for entraining a substance, and in particular, a gas phase substance, within an airflow to form a condensation aerosol is schematically illustrated in **Fig. 2A. Fig. 2A** shows another scheme for routing an airflow through a plurality of holes and across a surface of a structure. **Fig. 2A** shows a device having a first airway **30,** a second airway **31,** and a structure **32** separating first airway **30** and second airway **31.** Although structure **32** is shown as comprising two parts, e.g., as indicated by the thick and thin lines, structure **32** can comprise one part or multiple parts. Structure **32** includes a plurality of holes **39** which fluidly connect first airway **30** and second airway **31.** First airway **30** and second airway **31** are further defined by housing **34.** Housing **34** includes an air intake **35** to allow airflow **36** to enter second airway **31,** and an air outlet **37** to allow airflow **36** to exit the device. As shown in **Fig. 2A****,** first airway **30** and second airway **31** are further fluidly connected through holes and/or slots dimensioned to permit a greater, less than, or equal portion **38** of airflow **36** to pass into first airway **30,** compared to the portion of airflow the airflow that passes through plurality of holes **39.** The relative amounts of airflow to each airway can be altered to suit the desired purpose. In the same manner as described for Fig. **1B****,** the airflow through plurality of holes **39** as indicated by small arrows **33,** entrains the vaporized substance and the condensation particles **41** formed by condensation of the vaporized substance released from the plurality of supports **40** disposed on structure **32** within airflow **36.** Entrainment of condensation particles **41** within airflow **36** reduces deposition of the condensation particles **41** on downstream surfaces.

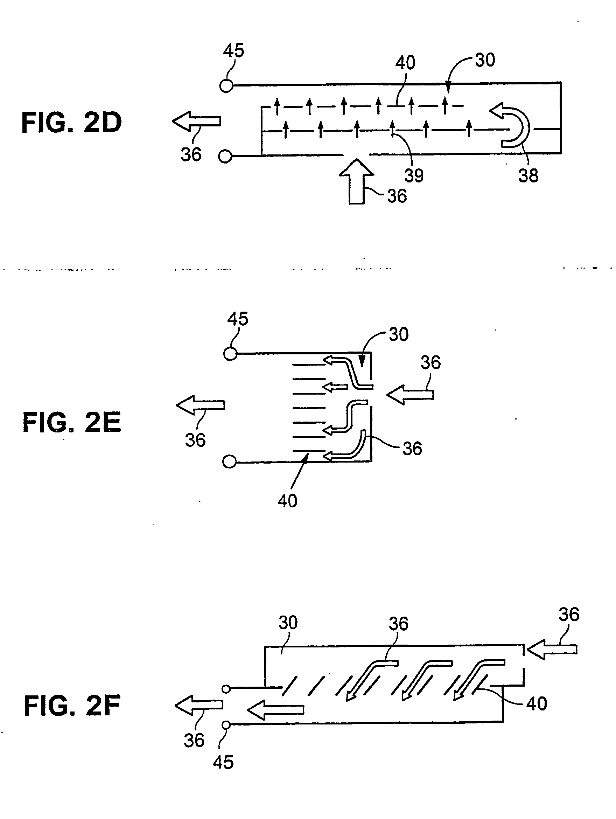

Another embodiment of a device for entraining a substance or condensation particles within an airflow is shown in **Fig. 2****B.** **Fig. 2B** shows a device similar to that of **Fig. 2A** wherein a second airflow **42,** which is a portion of airflow **36,** enters a third airway **43.** Second airflow **42** can then pass through the plurality of holes **39** to provide an airflow directed toward a plurality of supports **40** and the first airway **30.** The condensation particles **41** formed by vaporizing a substance disposed on the supports becomes entrained in airflow **36,** which includes airflows **38** and **42.**

In another embodiment, as shown in **Fig. 2C****,** a portion of first airflow **36** is directed through a porous element **44.** On passing through porous element **44,** this portion of airflow passes between supports **40** and directs the airflow toward first airway **30.** Porous element **44** can be fabricated from any material and have any pore size capable of distributing an appropriate portion of the air entering the device through the plurality of holes forming porous element **44.** For example, in certain embodiments, porous element **44** can be an open cell foam, a mesh, a fibrous material, a glass frit, a ceramic filter, a microporous element, and the like.

How effectively a substance is entrained within an airflow can at least in part depend on the proportion of rate of airflow across the surface of a support, R₁ to the rate of airflow through the plurality of openings, R₂. The appropriate proportion R₁ : R₂ for effectively entraining a substance within an airflow can depend on a number of factors such as the airflow velocity and the distance of the support from the center of the airflow. In certain embodiments, R₁ : R₂ can range from 80 : 20 to 20 :80 and in other embodiments can range from 60 : 40 to 40 : 60. The proportion R₁ : R₂ can be established by the relative areas of the holes through which the first an second airflows pass. For example, referring to **Fig 2A****,** a proportion of 60: 40 means that the relative area of hole/slot through which airflow **38** passes is 60 and the relative area of the plurality of holes **39** is 40.

Another embodiment of a device for entraining a substance in an airflow is shown in **Fig. 2D. Fig. 2D** shows airflow **36** entering the device. One portion of airflow **36 passes** through a plurality of holes **39** and across a plurality of supports **40.** A second portion of airflow **36** is diverted around the plurality of holes (shown on **Fig 2D** as **38).** The airflow portion that goes through the plurality of holes **39** and second airflow portion **38** recombine in first airway **30** and pass through mouthpiece 45 to exit the device.

In the embodiments shown in **Fig.1B** and **2A-D** by introducing air from below the supports redeposition of the vaporized substance or aerosol condensation particles is minimized.

Different arrangements of the supports with respect to the airflow through the device are shown in **Figs. 2E** and **2F****.** In **Fig. 2E****,** airflow **36** enters first airway **30.** Airflow **36** is routed over a plurality of supports **40** and recombines to pass through mouthpiece **45** to exit the device. In **Fig. 2F****,** airflow **36** entering first airway **30** passes over plurality of supports **40** to pass through mouthpiece **45** to exit the device.

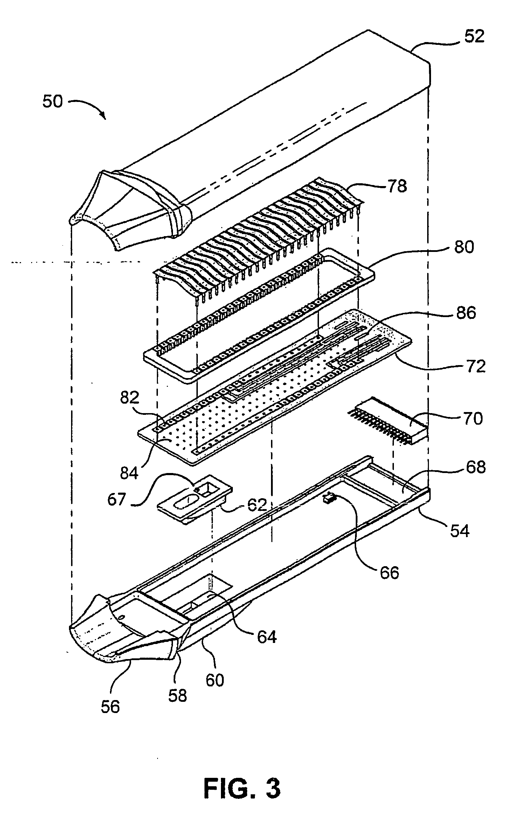

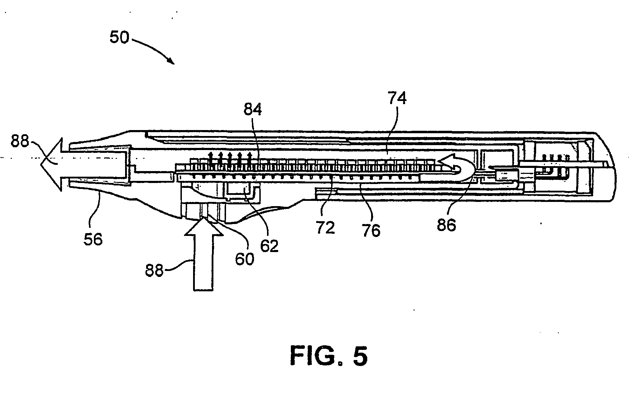

The concepts underlying the exemplary devices illustrated in **Figs.1B****,** **2A-2F** can be applied to devices for administering a condensation aerosol to a subject. A subject includes mammals and humans. A cartridge for administering multiple doses of a condensation aerosol to a subject which employs airflow through a plurality of holes to facilitate entrainment of a substance released from a support within an airflow is illustrated in **Fig. 3****.** An exploded assembly view of such a cartridge is shown in **Fig. 3** as part **50.** A cross-sectional view of an assembled cartridge is also illustrated in **Fig. 5****.**

**Fig. 3** shows an isometric assembly view of a cartridge capable of producing multiple doses of a substance for pulmonary administration. The cartridge **50** illustrated in **Fig. 3** comprises a first shell **52** and a second shell **54** which can be coupled to form a housing. When assembled, one end of first shell **52** and second shell **54** form a mouthpiece **56** for insertion in a subject's mouth. An air bypass hole **58** is located adjacent to mouthpiece **56** in second shell **54** to enable air to enter mouthpiece **56** when the rate of airflow generated by inhalation exceeds the rate of airflow controlled by an air inlet valve **62** entering the cartridge. The air inlet valve **62** can assist in minimizing any air flow variation from user to user. The rate of airflow in the housing can impact particle size and thus controlling air flow variation allows for more control over the particle size generated. The airbypass hole **58** allows for flexibility in that it allows the user to breath at a comfortable rate without upsetting the amount of air flow that moves through the housing and across the surface of the supports. For example, a person typically inhales at a flow rate ranging from 30 L/min to 100 L/min. A device, however, may have a flow rate of 6 L/min, which refers to the volume of air per time entering the device, being directed across the surface of the supports and emitted from the device, the excess airflow from the person will enter bypass hole **58.** Second shell **54** further comprises an air intake **60** (partially hidden). Air intake **60** includes air inlet valve **62** that fits into receptacle **64** of second shell **54.** As discussed above, air inlet valve **62** controls the airflow rate of the cartridge and can be any valve that can control the amount of air entering the device during a single inhalation by a user. Examples of appropriate valves include flapper valves (a flexible value that bends in response to a pressure differential), umbrella valves, reed valves, or flapping valves that bend in response to a pressure differential, and the like. The purpose of air inlet valve **62** is to control the amount of air entering the cartridge regardless of the total airflow rate during and among inhalations. The total airflow rate includes the airflow rate through air intake **60** and air inlet valve **62,** and the airflow rate through air bypass hole **58.**

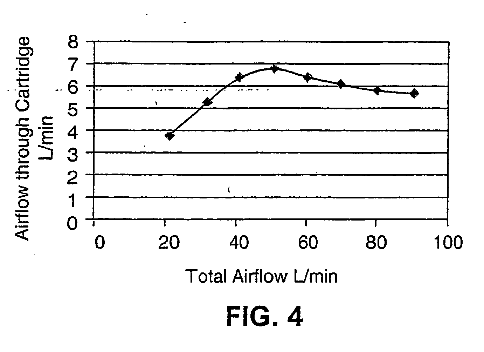

**Fig. 4** demonstrates that a simple flap valve can be used to control the airflow rate through the cartridge to about 6 L/min for total inhalation ranging from **20** L/min to 90 L/min. To generate the results presented in **Fig. 4****,** a cartridge was fitted with a flap valve and the airflow rate through the cartridge for various total airflow rates was measured. Thus, by using air inlet valve **62,** the airflow rate through the cartridge can be relatively independent of the airflow rate generated by an inhalation. As disclosed herein, flow control can be used to control the particle size and particle size distribution of the condensation aerosol emitted from the device. However, particle size and particle size distribution can be impacted by a number of additional factors including, for example, the substance, the vaporization temperature of the substance, the temperature of the airflow and the cross-sectional air of the airway. Thus, the airflow rate can be one of several parameters to be adjusted to produce a desired average particle size and particle size distribution. In certain embodiments, air control valve **62** can be designed to control the airflow through the cartridge between 4 L/min and 8 L/min. In certain embodiments, an airflow control valve can be activated electronically such that a signal provide by a transducer located within the airway can control the position of the valve, or passively, such as, for example, by a pressure differential between the airway and the exterior of the device. Additionally, the cross-sectional area of the airway can be adjusted to produce a desired average particle size and particle size distribution. In certain embodiments the cross-section area of the airway ranges from 0.5 cm² to 3 cm².

As shown in **Fig. 3****,** second shell **54** further includes a breath actuation mechanism **67.** Breath actuation mechanism **67** is electrically coupled to a remotely located controller (not shown) and can send a signal to the controller that interprets the data and activates the generation of a condensation aerosol when a certain preestablished airflow velocity is sensed. Breath actuation mechanism **67** can be, for example, a thermistor, which senses temperature in response to airflow. First shell **52** and second shell **54** also include a receptacle **68** for retaining electrical connector **70.** In addition, there can be a counter **66,** which identifies the number of supports that have not been actuated in that they have not been heated yet to vaporize the substance contained thereon.

When cartridge **50** is assembled, a structure **72** separates a first airway and a second airway. First airway **74** and second airway **76** are formed by structure **72** and the opposing inner walls of first and second shells **52, 54,** respectively, as shown in the cross-sectional view of the assembled cartridge illustrated in **Fig. 5****.** As shown in **Fig. 3****,** structure **72** is a printed circuit board enabling electrical connection between connector **70** and a plurality of electrically resistive heating elements **78.** Heating elements **78** are mounted on spacer **80** and soldered to interconnection lands **82** disposed on structure **72.** Spacer **80** can be a thermally insulating material such as, for example, a printed circuit board material.

As shown in **Fig. 3****,** structure **72** includes a plurality of holes **84** extending over most of the surface of structure **72.** Each of the holes **84** extends through the thickness of structure **72.** Structure **72** also includes a set of slots **86** near the end of structure **72** on which connector **70** is mounted. The number and dimensions of plurality of holes **84** and set of slots **86** determine the relative proportion of air which flows through the plurality of holes **84** and set of slots **86** when a subject inhales on mouthpiece **56.** As shown in **Fig. 5****,** when a subject inhales on mouthpiece **56** of cartridge **50,** an airflow **88** is generated such that air enters air intake **60,** the flow of air entering the device is controlled by air inlet valve **62** to enter second airway **76.** A first portion of airflow passes from second airway **76** through a set of slots **86** into first airway **74** to be inhaled by a subject. At the same time, a second portion of airflow passes through plurality of holes **84** and enters first airway **74** to be inhaled by the subject. The airflows passing through the plurality of holes **84** and the set of slots **86** combine to pass through mouthpiece **56** to exit the device.

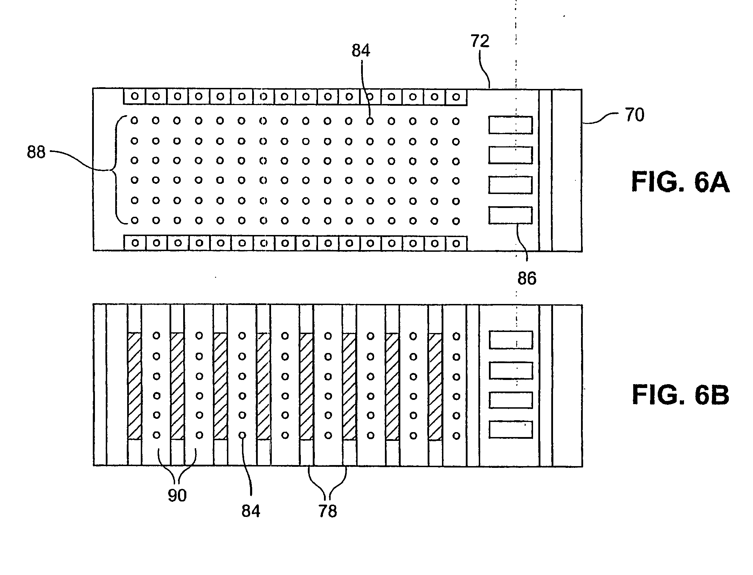

A top view showing the positioning of plurality of holes **84** and set of slots **86** with respect to plurality of supports **78** is shown in **Figs. 6A** and **6B. Fig. 6A** shows structure **72** comprising connector **70,** set of slots **86** and plurality of holes **84.** Set of slots **86** are shown as rectangular slots. However, set of slots **86** can have any number of openings, shapes, and/or dimensions as appropriate to cause a vaporized substance to become entrained within the airflow so as to form a condensation aerosol that exhibits appropriate properties for inhalation administration. Plurality of holes **84** is shown as comprising a regular array of round openings. However, plurality of holes **84** can have any number of openings, shapes, and/or dimensions as appropriate to cause a vaporized substance and condensation aerosol particles to be entrained within the airflow to form a condensation aerosol exhibiting appropriate properties for inhalation administration. For example, each row of holes **88** can instead be a narrow slot. Plurality of holes **84** can also be placed in a different arrangement over the surface of structure **72.**

As shown in **Fig. 6B****,** in certain embodiments, holes **84** can be positioned beneath gaps **90** between adjacent heating elements **78.** Air flowing from holes **84** through gaps **90** can direct a substance released from supports **78** into an airflow. In certain embodiments, at least some of the plurality of holes **84** can be located beneath at least some of the supports **78.**

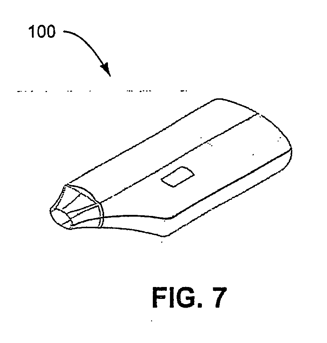

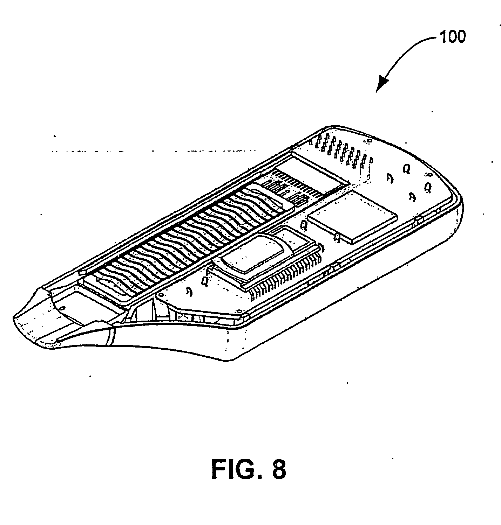

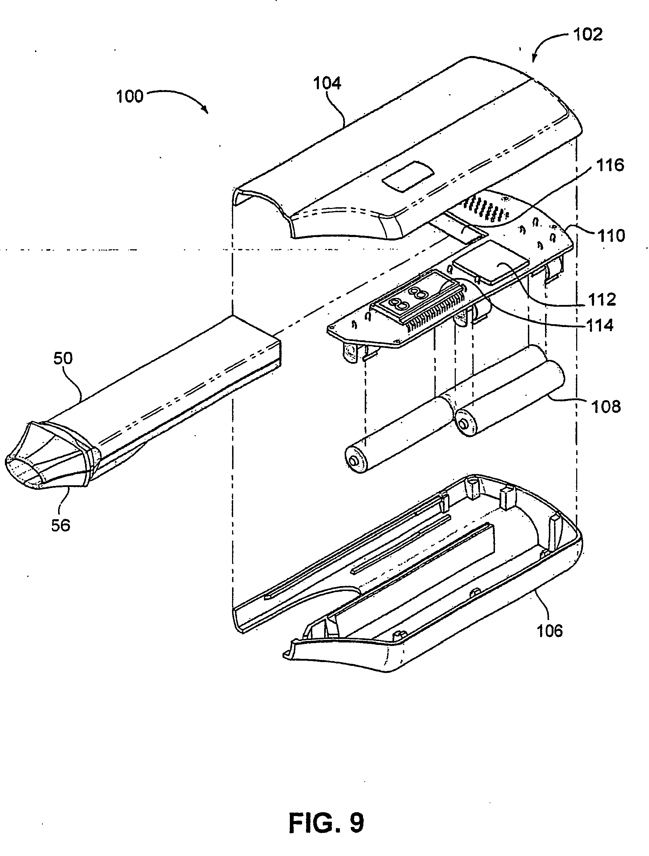

A cartridge as described in **Figs. 2-6** can be used in a condensation aerosol delivery device for the administration of a physiologically active substance to a subject. A solid view of an exemplary condensation aerosol delivery device **100** according to the disclosure is shown in **Fig. 7****.** An isometric view with the top of the device and the cartridge removed is shown in **Fig. 8****,** and an exploded isometric view of the condensation aerosol delivery device **100** is shown in **Fig. 9****.** Referring to **Fig. 9****,** the condensation aerosol delivery device **100** includes cartridge **50** and a dispensing unit **102.** As shown in **Fig. 9** cartridge **50** can be a separable unit. In certain embodiments, cartridge **50** can be an integral component of dispensing unit **102.** Dispensing unit **102** includes a first shell **104** and a second shell **106** which can be assembled to form the housing of dispensing unit **102.** As shown in **Fig. 9****,** dispensing unit **102** further includes a battery power source **108,** and a printed circuit board **110** incorporating a microprocessor controller **112,** a display **114,** and a connector **116** for connecting the dispensing unit with the cartridge and which also connects to controller **112** and power source **108** comprising three AAA batteries to cartridge **50.**

To deliver a condensation aerosol to a subject, the subject places mouthpiece **56** of condensation aerosol delivery device **100** into his or her mouth. The subject then inhales on mouthpiece **56** to generate an airflow as described herein. When a certain minimum airflow or a rate in change in airflow is sensed, the device is triggered. A signal from the airflow sensor is sent to the controller to cause the battery power source to connect to at least one support. As described herein, the supports can be, for example, electrically resistive heating elements. Heat produced by the electrically resistive heating element thermally vaporizes the substance disposed thereon. The vaporized substance condenses in the airflow to form condensation particles and hence, a condensation aerosol. As described herein, the airflow passing from beneath the heating element causes the substance vaporized from the heating element or the condensed aerosol particles to become entrained in the airflow as opposed to depositing on other supports prior to passing through the cartridge. The aerosol upon passing through the cartridge is subsequently inhaled by the subject. Activation of the condensation aerosol delivery device, generation of the condensation aerosol, and inhalation of the condensation aerosol can occur in a single breath. The inhaled condensation aerosol then enters the subject's respiratory tract where the condensation aerosol comprising the active substance can be deposited in the respiratory tract, and in particular the pulmonary alveoli, of the subject.

A device for generating a condensation aerosol can include at least one support and in certain embodiments, for example, as shown in **Figs. 2-5** and **8,** can include a piuraiity of supports. The supports can provide a surface and/or structure on which a substance to be released into an airflow can be disposed. In certain embodiments, the supports can be located at a side of the airway, for example on the surface of the structure, or can be located toward, near, or in the center of the airway. The shape and dimensions of the supports, and the material or materials forming the supports can be chosen to facilitate release of a substance disposed on the supports upon the application of energy, to minimize degradation of the substance during release, to cause rapid heating of the substance disposed thereon and/or to minimize the amount of energy used to release the substance.

Selection of the appropriate material for forming the support can also, at least in part, be determined by the source of energy used to release the substance from the support. For example, the source of energy used to release the substance can be mechanical, acoustic, radiation such as microwave, radio frequency or optical, and/or thermal. When the applied energy is absorbed directly by the substance, the support can be non-thermally conductive. For example, an optical source can be used to ablate and/or vaporize a substance disposed on a support. Alternatively, in certain embodiments, it can be more efficient or practical to heat a thermally conductive support which transfers thermal energy to the substance disposed thereon to release the substance from the support. In such embodiments, the support can be a thermally conductive material such as a metal, a metal alloy, a metal composite having more than one layer and/or composition, graphite, or the like. For example, in certain embodiments the metal can be stainless steel, copper, nickel, aluminum, gold, or silver, and can be plated with one or more of the foregoing materials or other metals. In some embodiments, the thickness of the plating of a metal layer on the metal can be within the range of between 0.001µm to 3 µm and in other embodiments.. In some embodiments, the support can be a semi-conducting material.

In certain embodiments, for example, where the condensation aerosol delivery device is designed for portable use with a battery power source, efficient energy use can be desirable. Minimization of the energy used to release a substance from a support can, at least in part, depend on the shape and dimensions of the support, the materials forming the support, and the placement of the support within the airway. In certain embodiments, the support can comprise an electrically resistive material such as a foil. In certain embodiments, the foil can be a stainless steel foil and can include a layer of one or more materials such as a gold layer to facilitate, for example, forming an electrical connection, and/or modifying the electrical properties such as the resistance of a portion of the foil. The appropriate dimensions for a foil can depend at least in part, on the desired resistance, the amount of substance disposed on the support, the amount of energy needed to vaporize the substance disposed on the support, and/or on mechanical stability considerations.

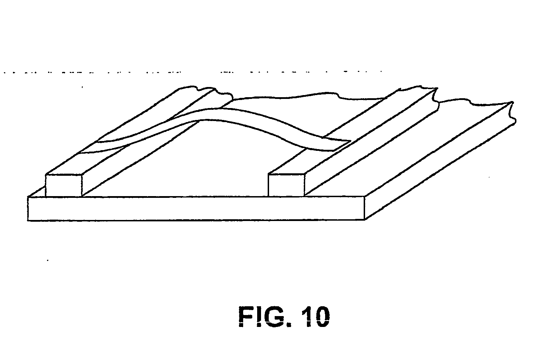

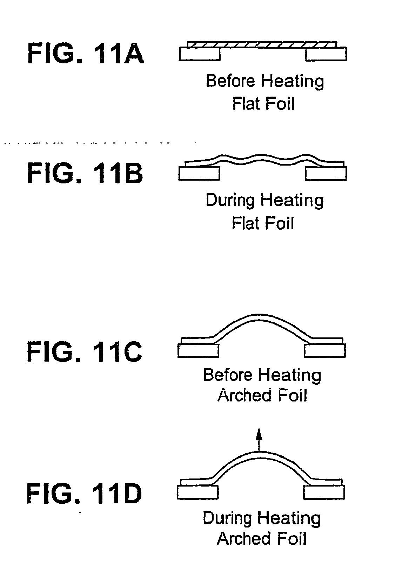

To maximize transfer of thermal energy produced by the support to the substance disposed thereon, it is desirable that a thermally conductive support be thermally isolated. Minimizing the contact area between the support and the connector helps to thermally isolate the support. As shown, for example, in Fig. 3, thermal isolation can be accomplished by suspending the support in the airflow above the surface of the structure by means of a spacer whereby the ends of the metal foil can be electrically connected to the power source. As shown in **Figs. 3****,** **8** and **10****,** in certain embodiments, the metal foil can be arched. During heating, thin foils can have a tendency to distort. This phenomenon is schematically illustrated in **Fig. 11****,** where a metal foil is shown suspended between two conductors. **Fig. 11(a)** shows a flat metal foil spanning two conductors. During heating, the flat metal foil can distort as shown schematically in **Fig. 11(b)****.** In a multiple dose condensation aerosol delivery device comprising several metal foil supports, such mechanical distortion of the foils can interact with the airflow to increase deposition of the condensation aerosol particles on downstream surfaces. To facilitate the accuracy and reproducibility of the amount of substance released upon firing from each support or heating element and transferred to recipient, it can be desirable that the airflow characteristics of the device be consistent for each actuation of the device. While distortion of a metal foil can be minimized by using thicker foils, efficient heating of the metal foils with minimum power consumption indicates the use of thin foils. It has been found that the mechanical stability of a metal foil can be improved by producing a slight arch in the foil. An example of an arched foil is shown in **Fig. 11(c)****.** During heating, the arched metal foil shown in **Fig. 11(c)** can exhibit a slight upward movement as indicated in **Fig. 11(d)****,** and following heating returns to approximately the same arched configuration as prior to heating. The arch can be formed a number of ways, such as, for example, but not limitation, assembly by placing the metal foil, or plurality of metal foils over an arched mandrel and bonding the ends to a platform. The metal foil can be too thin to take a permanent set, but can be held in slight compression to maintain the arch. The platform on which the arched metal foil is mounted can be for example, a spacer such as spacer **80** as shown in **Fig. 3****,** or can be structure **72** separating the first and second airways in embodiments where a spacer is not employed. In some embodiments of the invention, the height of the arch can ranges from 0.5 mm to 2 mm.

Particularly for portable, battery operated condensation aerosol delivery devices, it can be useful to minimize the amount of power used to vaporize a substance. Several characteristics of the metal foil can be chosen to facilitate the efficient thermal vaporization of a substance from a metal foil, including, but not limited to, the thickness of the metal foil, the impedance of the metal foil, and the ratio of the surface area to the thermal mass of the metal foil. In certain embodiments, the thickness of the metal foil can be less than 0.0254 cm (0.01 inches), in certain embodiments, less than 0.00254 cm (0.001 inches), and in certain embodiments, less than 0.00127 cm (0.0005 inches). To minimize power dissipation in the electrical circuit and thereby maximize power delivered to the heating element, it can be desirable that the impedance of the metal foil be closely matched to the impedance of the power source. For example, in certain embodiments, the difference between the impedance of the resistive heating element and the impedance of the power source can be less than 50% of the impedance of the power source, in certain embodiments, less than 10% of the impedance of the power source, and in certain embodiments, less than 2% of the impedance of the power source. To facilitate the efficient transfer of thermal energy produced by the resistive heating element to the substance disposed thereon, it can be useful to maximize the ratio of the surface area of the resistive heating element to the thermal mass of the resistive heating element. Accordingly, in certain embodiments the ratio of the surface area of the heating element to the thermal mass of the resistive heating element can be greater than 10 cm²/J/°C, in certain embodiments, greater than 100 cm²/J/°C, and in certain embodiments, greater than 500 cm²/J/°C.

Low ratios of the surface area of the heating element to the thermal mass of the resistive heating element can facilitate the transfer of heat to the substrate, and lead to rapid thermal vaporization of the substance. Rapid thermal vaporization of a substance can minimize thermal degradation of the substance during vaporization and thereby maximize the purity of the condensation aerosol formed therefrom. For example, in certain embodiments, the support, and in particular, a metal foil can be heated to a temperature of at least 250 °C in less than 500 msec, in certain embodiments, to a temperature of at least 250 °C in less than 250 msec, and in certain embodiments, to a temperature of at least 250 °C in less than 100 msec.

Efficient transfer of thermal energy produced by the resistive heating element to the substance disposed thereon can further be facilitated by the substance being disposed on the surface as a thin layer. For example, in certain embodiments, the thickness of the layer of substance can range from 0.01 µm to 50 µm, in certain embodiments, can range from 0.01 µm to 20 µm, and in certain embodiments, can range from 0.01 µm to 10 µm.

The amount of energy to thermally vaporize a substance can be minimized by, for example, using an electrically resistive heating element comprising a thin metal foil, closely matching the impedance of the electrically resistive heating element to the impedance of the power source, maximizing the ratio of the surface area of the resistive heating element to the thermal mass of the resistive heating element, and using a thin film of substance disposed on the heating element. By appropriate design and selection of at least the foregoing parameters, in certain embodiments, the amount of energy to vaporize a substance from a support can be less than 250 joules, in certain embodiments, less than 50 joules, and in certain embodiments, less than 10 joules. In more specific embodiments, the amount of energy to vaporize one mg of substance from a support can be less than 250 joules, in certain embodiments, less than 50 joules, and in certain embodiments, less than 10 joules.

The number of supports forming a condensation aerosol delivery device and/or cartridge is not particularly limited. For example, in certain embodiments, a cartridge or drug delivery device can comprise from 1 to 200 supports, in certain embodiments, from 1 to 50 supports, and in certain embodiments, from 1 to 25 supports, and in certain embodiments, from 1 to 10 supports.

The cartridge can be separable from the condensation aerosol delivery device. In such embodiments, a subject can use the delivery device, for example, to administer more than one physiologically active substance, or more than one dose of the same physiologically active substance by replacing one cartridge with another. Also, when all the doses in a particular cartridge are exhausted, the user can obtain and insert a new cartridge into the delivery device.

While certain embodiments of the present disclosure can comprise a single support, it is contemplated that embodiments comprising a plurality of supports can be particularly useful in, for example, providing a convenient method of delivering multiple doses of a physiologically active compound or drug over a period of time. The terms physiologically active compound and drug are used interchangeably herein. As used herein, a drug refers to a substance recognized in an official pharmacopoeia or formulary, and/or a substance intended for use in the diagnosis, cure, mitigation, treatment or prevention of disease where disease refers to any disease, disorder, condition, symptom or indication. In such embodiments, the substance disposed on at least one support can comprise a therapeutically effective amount of a drug. For example, a therapeutically effective amount or dose of a drug can be disposed on a single support, on each of multiple supports, or on more than one support. In certain embodiments of a condensation aerosol delivery device, the same amount of physiologically active compound can be disposed on each support. In certain embodiments, different amounts of a physiologically active compound can be disposed on each of the plurality of supports, or a certain amount of active compound can be disposed on several supports, and a different amount of active compound on several other supports. Having different amounts of a drug on different supports can be useful in effecting treatment regimens where administering a variable amount of drug during a period of time is useful.

In certain embodiments, where the active compound disposed on several supports is an abusable substance, a second compound comprising an agonist can be disposed on one or more other supports. "Abusable substance" refers to a substance that can be improperly used, for example, by administering more than a prescribed or intended dosage, or by altering the route of administration from the intended route. For example, an opioid analgesic can be abused by using the opioid analgesic to elicit a euphoric effect, rather than therapeutically for the treatment of pain. Abusable substances include substances regulated by a regulatory agency focused on preventing drug abuse, such as, for example, the United States Drug Enforcement Agency (DEA). In certain embodiments, an abusable substance can be a substance listed on DEA schedule II, III, IV, or V. The second compound is a chemical compound that can act to reduce or to counteract the physiological activity and/or pharmacological effects of another chemical substance. Having both an abusable substance and a second compound capable of counteracting the effects of the abusable substance in the same device will complicate the ability of an abuser to selectively remove the abusable substance from heating elements. Proper use of the device would only allow the abusable substance to be activated in prescribed doses.

A substance to be released can be disposed on at least one surface of a support. For example, the substance can be disposed on the surface facing the center of the first airway and/or toward the part of the airflow where the velocity is highest. The substance can be applied to a surface of a support by any appropriate method and can depend at least in part on the physical properties of the substance and the final thickness of the layer to be applied. In certain embodiments, methods of applying a substance to a support include, but are not limited to, brushing, dip coating, spray coating, screen printing, roller coating, inkjet printing, vapor-phase deposition, spin coating, and the like. In certain embodiments, the substance can be prepared as a solution comprising at least one solvent and applied to a support. In certain embodiments, a solvent can comprise a volatile solvent such as acetone, or isopropanol. In certain embodiments, the substance can be applied to a support as a melt. In certain embodiments, a substance can be applied to a film having a release coating and transferred to a support. For substances that are liquid at room temperature, thickening agents can be admixed with the substance to produce a viscous composition comprising the substance that can be applied to a support by any appropriate method, including those described herein. In certain embodiments, a layer of substance can be formed during a single application or can be formed during repeated applications to increase the final thickness of the layer. In other embodiments, the substance can be applied on more than one surface of the support.

In certain embodiments, more than one active compound can be disposed on one or more of the plurality of supports. For example, a first active compound can be disposed on certain supports, and a second active compound can be disposed on other supports, and in certain embodiments, a composition comprising a first active compound and a second active compound can be disposed on one or more supports.

A dose can correspond to the amount of active compound released from a single support, or the amount of active compound released from more than one support. A dose or dosage as used herein refers to the amount of substance released during a single activation of a condensation aerosol delivery device. A dose can comprise a therapeutically amount of a physiologically active compound, meaning that the dose provides effective treatment of a condition and/or disease in a patient. The therapeutically effective amount of a physiologically active compound can vary from compound to compound, from subject to subject, and can depend upon factors such as the condition of the subject.

In certain embodiments, a substance disposed on at least one support can comprise a therapeutically effective amount of at least one physiologically active compound or drug. A therapeutically effective amount refers to an amount sufficient to effect treatment when administered to a patient or user in need of treatment. Treating or treatment of any disease, condition, or disorder refers to arresting or ameliorating a disease, condition or disorder, reducing the risk of acquiring a disease, condition or disorder, reducing the development of a disease, condition or disorder or at least one of the clinical symptoms of the disease, condition or disorder, or reducing the risk of developing a disease, condition or disorder or at least one of the clinical symptoms of a disease or disorder. Treating or treatment also refers to inhibiting the disease, condition or disorder, either physically, e.g. stabilization of a discernible symptom, physiologically, e.g., stabilization of a physical parameter, or both, and inhibiting at least one physical parameter that may not be discernible to the patient. Further, treating or treatment refers to delaying the onset of the disease, condition or disorder or at least symptoms thereof in a patient which may be exposed to or predisposed to a disease, condition or disorder even though that patient does not yet experience or display symptoms of the disease, condition or disorder. In certain embodiments, the amount of substance disposed on a support can be less than 100 micrograms, in certain embodiments, less than 250 micrograms, in certain embodiments, less than 500 micrograms, and in certain embodiments, less than 1,000 micrograms.

When delivering a pharmaceutical compound to a subject, the amount of substance that is vaporized off the surface is important. Consistency of delivery of the compound is also critical. In certain embodiments, at least 80% of the amount of material disposed on each support passes through the outlet of the device for deliver to the subject, in other embodiments, at least 90% passes through the outlet, and in other embodiments, at least 98% passes through the outlet.

In certain embodiments, a substance can comprise a pharmaceutical compound. In certain embodiments, the substance can comprise a therapeutic compound or a non-therapeutic compound. A non-therapeutic compound refers to a compound that can be used for recreational, experimental, or pre-clinical purposes. Classes of drugs that can be used include, but are not limited to, anesthetics, anticonvulsants, antidepressants, antidiabetic agents, antidotes, antiemetics, antihistamines, anti-infective agents, antineoplastics, antiparkinsonian drugs, antirheumatic agents, antipsychotics, anxiolytics, appetite stimulants and suppressants, blood modifiers, cardiovascular agents, central nervous system stimulants, drugs for Alzheimer's disease management, drugs for cystic fibrosis management, diagnostics, dietary supplements, drugs for erectile dysfunction, gastrointestinal agents, hormones, drugs for the treatment of alcoholism, drugs for the treatment of addiction, immunosuppressives, mast cell stabilizers, migraine preparations, motion sickness products, drugs for multiple sclerosis management, muscle relaxants, nonsteroidal antiinflammatories, opioids, other analgesics and stimulants, ophthalmic preparations, osteoporosis preparations, prostaglandins, respiratory agents, sedatives and hypnotics, skin and mucous membrane agents, smoking cessation aids, Tourette's syndrome agents, urinary tract agents, and vertigo agents.

Examples of pharmaceutical compounds include fluticasone propionate, clonidine, triazolam, albuterol, ciclesonide, fentanyl, terbutaline, flumazenil, triamcinolone acetonide, flunisolide, ropinirole, alprazolam, buprenorphine, hyoscyamine, atropine, pramipexole, bumetanide, flunitrazepam, oxymorphone, colchicine, apomorphine HCl, granisetron, pergolide, nicotine, loperamide, azatadine,naratriptan, clemastine, benztropine, ibutilide, butorphanol, fluphenazine, estradiol-17-heptanoate, zolmitriptan, metaproterenol, scopolamine, diazepam, tolterodine, estazolam, haloperidol, carbinoxamine, estradiol, hydromorphone, bromazepam, perphenazine, midazolam, methadone, frovatriptan, eletriptan, testosterone, melatonin, galanthamine, cyproheptadine, bropheniramine, and chlorpheniramine. In certain embodiments, the compound is chosen from alprazolam, buprenorphine, clonindine, fentanyl, midazolam, pramipexole, ropinirole, and triazolam. In certain embodiments, the compound is chosen from a compound for the treatment of pain. In certain embodiments, the compound for the treatment of pain is fentanyl.

In certain embodiments, a drug can further comprise substances to enhance, modulate and/or control release, aerosol formation, intrapulmonary delivery, therapeutic efficacy, therapeutic potency, stability, and the like. For example, to enhance therapeutic efficacy a drug can be co-administered with one or more active agents to increase the absorption and/or diffusion of the first drug through the pulmonary alveoli, or to inhibit degradation of the drug in the systemic circulation. In certain embodiments, a drug can be co-administered with active agents having pharmacological effects that enhance the therapeutic efficacy of the drug. In certain embodiments, a drug can comprise compounds that can be used in the treatment of one or more diseases, conditions, or disorders. In certain embodiments, a drug can comprise more than one compound for treating one disease, condition, or disorder, or for treating more than one disease, condition, or disorder.

In certain embodiments, the substance can comprise one or more pharmaceutically acceptable carriers, adjuvants, and/or excipients. Pharmaceutically acceptable refers to approved or approvable by a regulatory agency of the Federal or a state government or listed in the U.S Pharmacopoeia or other generally recognized pharmacopoeia for use in animals, and more particularly in humans.

In general, substances useful in embodiments of the disclosure can exhibit a heat of vaporization less than about 150 kJoules/mol.

Not only can the amount of compound forming a dose be impacted by deposition of aerosol particles on the device and other supports in the device, but the amount of compound forming a dose can be reduced by degradation of the active agent during release from the support. While it will be recognized that the extent and dynamics ofthermal degradation can at least in part depend on a particular compound, in certain embodiments, thermal degradation can be minimized by rapidly heating the substance to a temperature sufficient to vaporize and/or sublime the active substance. In certain embodiments, the support or heating element can be heated to a temperature of at least 250 °C in less than 500 msec, in certain embodiments, to a temperature of at least 250 °C in less than 250 msec, and in certain embodiments, to a temperature of at least 250 °C in less than 100 msec.

In certain embodiments, rapid vaporization of a layer of substance can occur with minimal thermal decomposition of the substance, to produce a condensation aerosol exhibiting high purity of the substance. For example, in certain embodiments, less than 10% of the substance is decomposed during thermal vaporization resulting in a condensation aerosol with at least 90% purity and in certain embodiments, less than 5% of the substance is decomposed during thermal vaporization resulting in a condensation aerosol with at least 95% purity, and in other embodiments, less than 2% of the substance is decomposed during thermal vaporization resulting in a condensation aerosol with at least 98% purity.

For administration of a compound, the size of the particulates of the compound comprising the aerosol can be within a range appropriate for intrapulmonary delivery. Without being limited by theory, an aerosol having a mass median aerodynamic diameter ("MMAD") ranging from 1 µm to 3 µm, and ranging from 0.01 µm to 0.10 µm are recognized as optimal for intrapulmonary delivery of pharmaceutical compounds. Aerosols characterized by a MMAD ranging from 1 µm to 3 µm can deposit on alveoli walls through gravitational settling and can be absorbed into the systemic circulation, while aerosols characterized by a MMAD ranging from about 0.01 µm to 0.10 µm can also be deposited on the alveoli walls through diffusion. Aerosols characterized by a MMAD ranging from 0.15 µm to 1 µm are generally exhaled. Thus, in certain embodiments, aerosols produced using devices and methods of producing an aerosol can having a MMAD ranging from 0.01 µm to 5 µm, in certain embodiments, a MMAD ranging from 0.05 µm to 3 µm, in certain embodiments, a MMAD ranging from 1 µm to 3 µm and in certain embodiments, a MMAD ranging from 0.01 µm to 0. 1 µm. In certain embodiments, aerosols suitable for intrapulmonary delivery of pharmaceutical compounds can further be characterized by the geometric standard deviation of the log-normal particle size distribution. In certain embodiments, aerosols produced using the devices and methods of producing an aerosol comprise a geometric standard deviation of the log-normal particle size distribution of less than 3, in certain embodiments, less than 2.5, and in certain embodiments, less than 2.

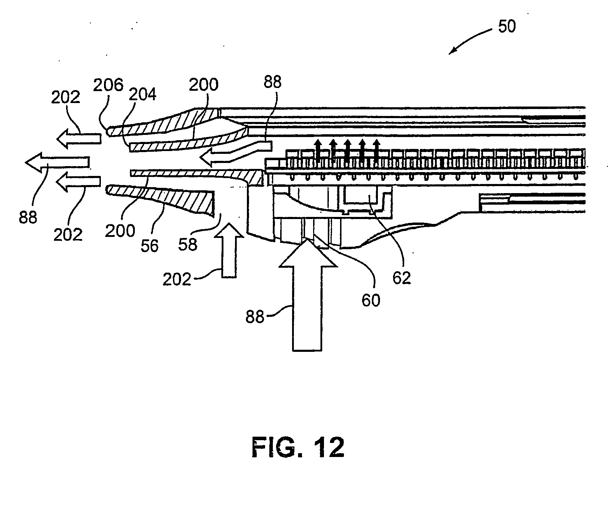

In certain embodiments, a cartridge can include a part disposed in the mouthpiece to control the airflow exiting the device. A partial section view of the cartridge cross-section of **Fig. 5** is shown in **Fig. 12. Fig. 12** shows the front section of cartridge **50,** further including an air routing part **200** disposed within the mouthpiece **56.** The airflow **88** entering air intake **60,** and air inlet valve **62** passes through the internal airways to entrain a condensation aerosol particles, and passes through the orifice defined by air routing part **200** to be emitted from the device. Bypass airflow **202** enters bypass opening **58** and is diverted around the outside of air routing part **200.** The front **204** of air routing part **200** extends to near the tip **206** of mouthpiece **56.** The use of air routing part **200** can be useful in maintaining smooth airflow through the device and facilitating control of the condensation aerosol particle size.

An embodiment of a condensation aerosol delivery device is the portable electric multi-dose drug delivery systems discussed herein, and illustrated in **Figs. 7** to **9****.** The electric multi-dose drug delivery system is designed to produce and deliver a therapeutic condensation aerosol into the respiratory tract, and in particular to the pulmonary pathway, of a subject. As discussed herein, the condensation aerosol delivery device includes two subsystems, referred to as the cartridge and the dispensing unit. Both the cartridge and the dispensing unit incorporate several electronic features which facilitate the portability, safety, versatility, and convenience of the delivery device. As disclosed herein, the cartridge includes the therapeutic drug in individual doses, and electronics to sense airflow generated by the subject's inhalation. The dispensing unit includes a battery power source, and a microcontroller that controls the drug vaporization process, and can include a number of communication functions. Such communication functions include, but are not limited to, cartridge identification, dose identification, abuse prevention functions, use monitoring, and dose control.

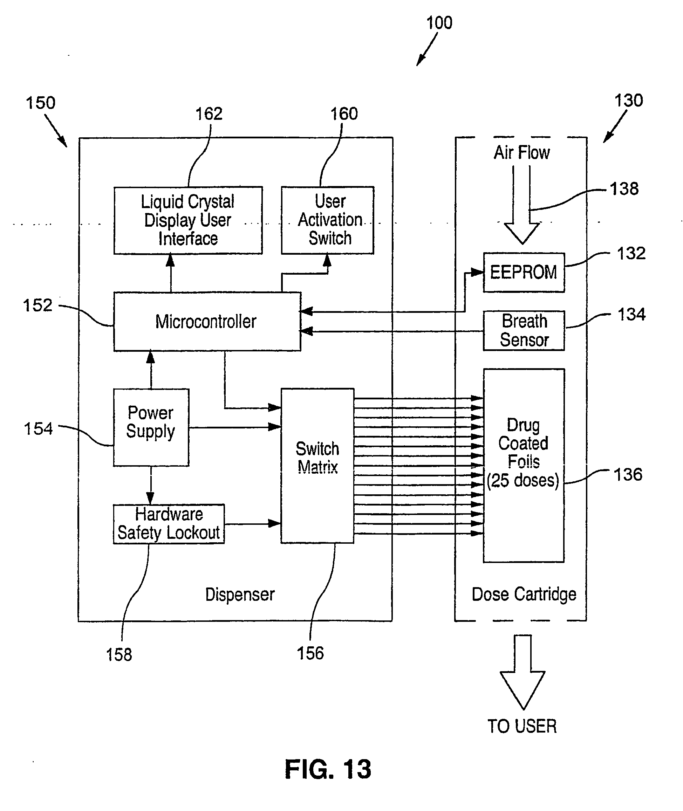

A functional block diagram of the electronics for an exemplary embodiment of an electric multi-dose condensation aerosol delivery device **100** is shown in **Fig. 13. Fig. 13** shows a cartridge **130** comprising an EEPROM **132,** a breath sensor **134,** and twenty-five drug coated metal foils **136.** EEPROM **132** can include, for example, an identifying serial number for the cartridge, a manufacturing date, and/or additional identification and control information, and monitors the number of doses remaining in the cartridge. EEPROM **132** is electrically connected to microcontroller **152** contained in the dispensing unit **150.** Microcontroller **152** can read or write to EEPROM **132** to update and record the data stored therein. EEPROM **132** need not require power to maintain the data. Breath actuation sensor **134** includes circuitry for detection of airflow, and is electrically connected to microcontroller **152.** The circuitry can comprise two temperature sensing devices such as thermistors, one of which is heated. Air flowing across the heated sensor **134** is transduced as a change in voltage, which is monitored by microcontroller **152.** When a certain minimum velocity of airflow **138** is sensed, microcontroller **152** connects power source **154** to at least one of resistive metal foils **136** to effect vaporization of the drug disposed thereon. Plurality of drug coated foils **136** are electrically connected to a switch matrix **156** which is controlled by microcontroller **152.** As disclosed herein, plurality of drug coated foils **136** can be selectively heated by passing a current through the foils to vaporize the drug coating to form a condensation aerosol in airflow **138.**

As shown in **Fig. 13****,** dispensing unit **150** includes microcontroller **152,** power source **154,** switch matrix **156,** a hardware safety lock-out mechanism **158,** a user-activated switch **160,** and a liquid crystal display user interface **162.** Microcontroller **152** incorporates embedded software and controls operation of the condensation aerosol delivery device. When not operating, microcontroller **152** is maintained in a sleep mode to conserve power consumption. Upon momentary depression of user activation switch **160,** microcontroller **152** becomes operational. In certain embodiments, microcontroller **152** can also be activated by inserting a cartridge into the delivery device. Microcontroller **152** can then check for the presence of cartridge **130,** and if present, microcontroller **152** reads EEPROM **132** to determine whether the serial number of cartridge **130** matches the serial number stored in the controller, and calculates the number of unused doses contained on drug coated foils **136** remaining in cartridge **130.** A purpose of matching the cartridge and dispensing unit serial number can be to personalize individual cartridges **130** and dispensing unit **150** to an individual patient. Personalization can be programmed using the embedded software by a health care provider to facilitate and personalize a patient's treatment regimen, and to reduce the potential for abuse by preventing a particular cartridge from being used in a dispensing unit having a different serial number. Upon verification of the parameters, microcontroller **152** updates display **162** with, for example, the number of doses remaining in cartridge **130,** and waits for an activation signal from breath sensor **134.** When a patient establishes a sufficient airflow in cartridge **130** by inhaling on the cartridge mouthpiece, microcontroller **152** connects power source **154,** through switch matrix **156,** to one or more of drug coated foils **136** to release the drug to form a condensation aerosol comprising the drug in airflow **138** of cartridge **130** that is inhaled by the patient. Microcontroller **152** is electrically connected to switch matrix **156,** and can connect one or more of drug-coated foils **136** to power source **154** at a given time. In certain embodiments, microcontroller **152** can connect one or more drug coated foils **136** to power source **154** sequentially, randomly, or in a predetermined order. Following actuation to deliver a dose to the patient, microcontroller **152** can enter a lockout period in which a subsequent dose cannot be released until the lockout period expires. Microcontroller **152** can enter a sleep mode to conserve power until manually activated by depressing user activation switch **160,** inserting a cartridge in the device, and/or removing a cartridge.

Display **162** is an electronic display which can inform a user of the current state of the device, e.g., whether the device is in the sleep or activated mode, and the number of unused doses remaining in the cartridge. User activated switch **160** is a momentary push button switch that when depressed activates the system from the sleep mode. Power source **154** comprises three alkaline primary cells that are used to power the system including providing the power necessary to vaporize the drug disposed on metal foils **136.** Switch matrix **156** can be an array of MOSFET switches under control of the microcontroller that couple power from power source **154** to the appropriate drug coated foils **136.** Hardware safety lockout **158** is a redundant, software-independent system that can prevent more than one dose from being delivered at a time and/or prevent a second dose from being delivered before the end of the lockout period. Hardware safety lockout **158** provides a redundant safety mechanism in the event of software malfunction.

In certain embodiments, the device is such that the total airflow passing through the outlet ranges from 10 liters/min to 100 liters/min. In other embodiments, the total airflow passing though the outlet ranges from 20 liters/min to 90 liters/min.

In certain embodiments of the device, the airflow rate through the inlet is less than 100 L/min. In other embodiments, the airflow rate through the inlet is less than 50 liters/min. In yet other embodiments, the airflow rate through the inlet is less than 25 liters/min; and in still other embodiments, the airflow rate through the inlet is less than 10 liters/min.

It should also be evident from the various embodiments disclosed herein that many parameters can be selected and/or adjusted to provide a condensation aerosol delivery device, and in particular an electric condensation aerosol delivery device capable of delivering multiple doses of a physiologically active substance to a patient with each dose being delivered during a single inhalation. It will be appreciated that at least some of the parameters are interactive, and that the multiple parameters can be adjusted by routine optimization procedures to generate a condensation aerosol comprising a dose of a particular physiologically active substance. As discussed herein, such parameters include, but are not limited to the properties of a particular substance, e.g., heat of vaporization, the quantity of substance comprising a dose, the thickness of the layer disposed on the support, the thickness of the heating element, the ratio of the surface area of the heating element to the thermal mass of the resistive heating element, and the airflow.

### Examples

Embodiments of the present disclosure can be further defined by reference to the following examples, which describe in detail certain embodiments of the present disclosure.

### Example 1

### Electric Multi-Dose Condensation Aerosol Delivery Device

Electric multiple dose condensation aerosol delivery devices as shown in **Figs. 2-5** were fabricated. The two halves forming the housing of the cartridge were molded from either acrylonitrile-butadiene-styrene or polycarbonate. The structure separating the first and second airways was fabricated from 0.08128 cm (0.032-inch) thick FR4 printed circuit board material. When assembled, the circuit board and the walls of the cartridge define a 8.89 cm (3.5 inch) long first airway having a cross-sectional area of 1.5 cm², and a 7.62 cm (3.0 inch) long second airway having a cross-sectional area of 1.5 cm². The total resistance through the cartridge was 0.07 sqrt(cm-H₂O)/L/min at a total airflow rate of 20 L/min and 0.09 sqrt(cm-H₂O)/L/min at 90 L/min. The flow valve was designed to control the flow between 4 L/min and 8 L/min for a total flow rate ranging from 20 L/min to 90 L/min (see Fig. 4). Circuit boards used to separate the first and second airways were fabricated having different arrangements and dimensions of holes. In a certain exemplary embodiment, the plurality of holes beneath the metal foils comprised an array of 100 round holes situated beneath the gaps between adjacent metal foils. Sixty percent of the airflow entering the air control valve passed through a series of slots and across the heating elements in the first airway. Forty percent of the airflow passed through the plurality of holes in the circuit board and was directed toward the heating elements and the center of the first airway.

The device incorporated 25 supports. The supports were fabricated from 0.00127 cm (0.0005 inch) thick stainless steel foils having a surface area of 0.2 cm² and mounted in an arched configuration to minimize distortion during heating. Fifty µg of fentanyl was deposited on the surface of each foil by spray coating from a solution comprising either isopropyl alcohol, acetone, or acetonitrile. The 50 µg layer of fentanyl was 3 µm thick. The resistance of the metal foils on which the fentanyl was deposited was 0.4 Ω, the ratio of the surface area of the metal foil to the thermal mass of the heating foil was 47 cm²/J/C. Either three AAA batteries or a Hewlett Packard 6002A DC power supply were used, depending on the experiment conducted, to provided 1.7 joules of energy to the heating element to vaporize the 50 µg of fentanyl.

### Example 2

### Aerosol Particle Size Measurement

The size of aerosol particles can impact the therapeutic efficacy of a pharmaceutical administered by inhalation. For example, aerosols having a particle size ranging from 0.01 µm to 3 µm are considered optimal for pulmonary delivery. In addition to the dynamics of aerosols during inhalation, it can be important that a condensation aerosol delivery device generate a consistent and reproducible particle size distribution. Aerosol particle size can be characterized by the mass median aerodynamic diameter (MMAD) of the aerosol. MMAD refers to the median of the distribution of particle sizes forming the aerosol.

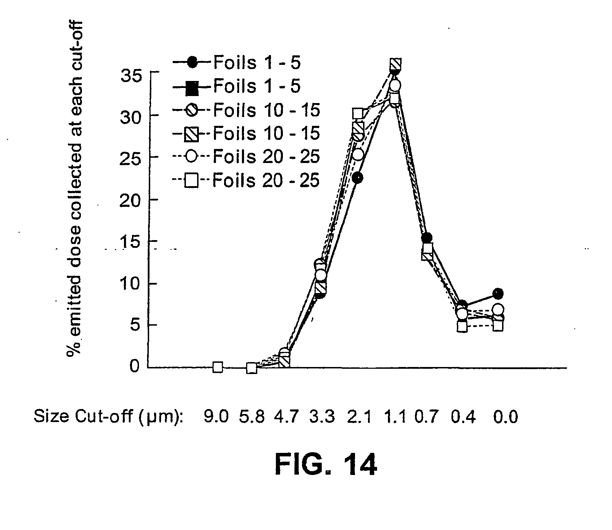

Aerosol particle size distributions for condensation aerosols formed using the condensation aerosol delivery device described in Example 1 are presented in **Fig. 14****.** Each foil of a 25-foil cartridge contained 50 µg of fentanyl as a 3 µm thick layer. A single foil was heated to a peak temperature of 400 °C within 350 msec in a 6 L/min airflow. The particle size distribution of the aerosol emitted from the device was measured by the Anderson Impaction method using an eight stage Cascade Impactor Series 20-800 Mark II (Anderson, Copley Scientific, Nottingham, UK). The particle size distribution for two replicates from each of front foils (1-5), middle foils (10-15) and back foils (20-25) (closest to the mouthpiece) are presented in **Fig. 14****.** The particle size distribution of the aerosol from each foil is consistent, exhibiting a range of particle size from about 5.8 µm to about 0 µm with a MMAD of 1.8 µm, and a geometric standard deviation (GSD) of 1.7 µm.

### Example 3

### Effect of Airflow on Particle Size

The airflow in a condensation aerosol delivery device as described in Example 1 was adjusted and the particle size of five emitted doses measured using the Anderson impaction method. The airflow volume was increased from 4 L/min to 8 L/min to increasing the airflow velocity from 1 m/sec to 2 m/sec. In tests 1, 2, and 4, a bypass air routing part was inserted into the mouthpiece section of the cartridge (to get the total airflow up to 28.3 L/min for the Andersen impactor to function properly) such that the bypass air and the airflow containing the condensation aerosol joined just prior to entering the impactor. In test 3, however, bypass air was introduced into the outgoing airflow immediately after passing over the heating elements. The results are presented in Table 1.

**TABLE 1 Effect of Airflow Rate on Aerosol Particle Size**

| | **Test 1** | **Test 2** | **Test 3** | **Test 4** |
|---|---|---|---|---|
| Airflow Rate (L/min) | 4 | 6 | 6 | 8 |
| Airflow Velocity (m/sec) | 1 | 1.5 | 1.5 | 2 |
| Percent Recovery | 83 | 90 | 86 | 90 |
| Emitted Dose (µg) | 208 | 225 | 216 | 224 |
| MMAD (µm) | 2.53 | 1.88 | 1.37 | 1.25 |
| GSD | 1.99 | 2.09 | 2.36 | 2.10 |
| FPF (1-3.5 µm) (%) | 56 | 61 | 60 | 58 |
| Fraction 0-2 µm (%) | 37 | 53 | 69 | 76 |
| Fraction < 5 µm (%) | 91 | 98 | 100 | 100 |

### Example 4

### Stability of Fentanyl In Multi-Dose Device

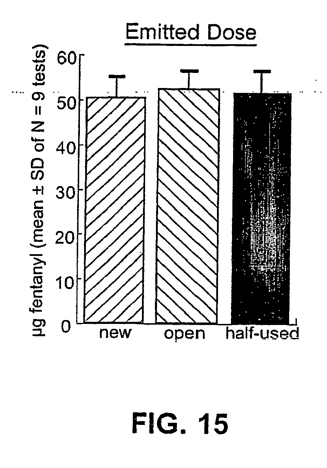

The stability of fentanyl in multi-dose condensation aerosol delivery devices was determined by measuring the amount and purity of fentanyl in an emitted dose for a newly manufactured cartridge (diagonal lines), an unused cartridge that was stored at room temperature for 7 days (cross-hatch), and a cartridge that was used to emit 10 doses and then stored at room temperature for 7 days (solid). The results are presented in **Fig. 15****.**

### Example 5

### Temperature Profile of Heating Element

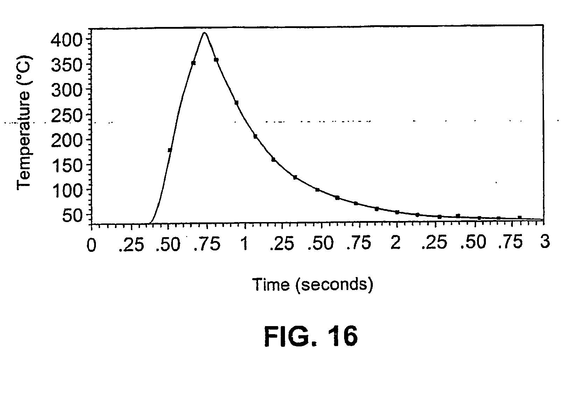

Three AAA batteries provided 1.7 joules of energy to a 0.00127 cm (0.0005 inch) thick stainless steel foil on which 50 µg of fentanyl was deposited. The airflow velocity was 1 m/sec corresponding to an airflow rate of 4 L/min. As shown in Fig. 16, the temperature of the foil increased to a temperature of about 200 °C within 50 msec, a maximum temperature of 400 °C within 284 msec, and returned to room temperature within 1.5 sec after reaching maximum temperature.

### Example 6

### Temperature Uniformity Measurements

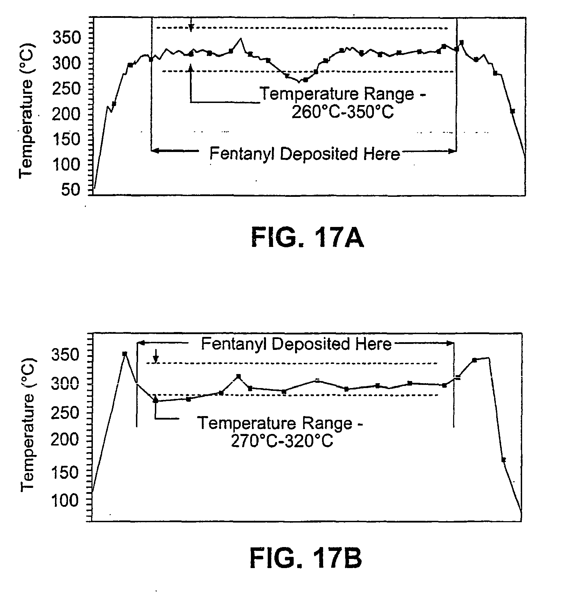

The temperature uniformity of a foil having a thin layer of 50 µg of fentanyl was measured during heating. The results are shown in **Figs. 17A and 17B****.**

### Example 8

### Effect of Second Airflow on Aerosol Particle Deposition

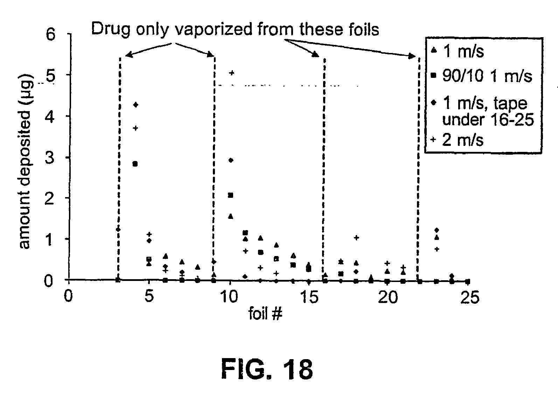

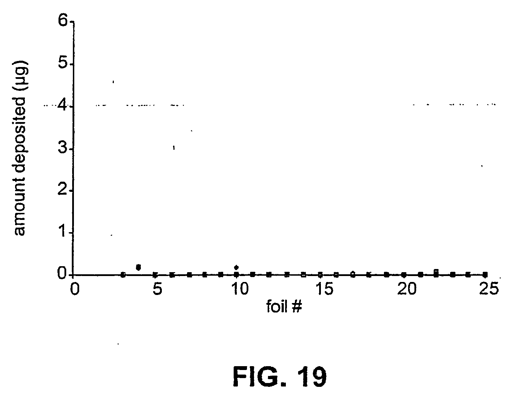

The effects of the airflow in a cartridge on the deposition of the aerosol particles on downstream surfaces is demonstrated in **Figs. 18** and **19****.** The results presented in **Fig. 18** were obtained using a cartridge as described in Example 1 with the exception that there was no circuit board separating the first and second airways and flow was controlled by flow meters instead of a flow valve. The heating elements were supported at the edges only and there was no flow control between the first and second airways; the amount of air entering the first and second airways was controlled by flow meters at the inlet to each airway. For the *1 m*/*s* and 2 *m*/*s* examples in **Fig. 18** the first and second airways were separated by a piece of tape to test aerosol particle deposition when all the airflow passed over the top of the heating elements. In the *90*/*10 1 m*/*s* example, in contrast, the tape was removed and the flow meters were set such that 90% of the inlet airflow entered through the first airway and 10% entered through the second airway. The air that entered through the second airway had to flow through the gaps between the heating elements to reach the airway outlet. Finally, in the *1 m*/*s, tape under 16-25* case a piece of tape was placed below heating elements 16-25 and again the flow meters were set such that 90% of the inlet airflow entered through the first airway and 10% entered through the second airway. The tape was intended to increase the amount of air flowing up past heating elements 1-15. In each experiment heating elements 3, 9, 16, and 22 contained a 3 µm thick layer of 50 µg of fentanyl from which fentanyl was vaporized, with the downstream elements fired before the upstream elements so that any deposited aerosol particles would not be revaporized. As shown in **Fig. 18****,** for each of these conditions up to about 5 µg of fentanyl was deposited on each downstream heating element.

**Fig.19** shows the results from three tests conducted using the same airway as described above for the results in **Fig. 18****.** In these tests, however, the first and second airways were separated by a thin piece of foam placed directly below the heating elements and the flow meters were set such that 50% of the inlet airflow entered through the first airway and 50% entered through the second airway. The foam created a pressure drop between the first and second airway, evenly distributing the flow from the second airway past each heating element and into the center of the first airway. In these experiments 50 µg of fentanyl were vaporized from each of the 25 heating elements (in contrast to the experiments from Fig. 18 where fentanyl was only vaporized from 4 heating elements) from downstream heating element 25 to upstream heating element 1, and essentially no fentanyl was deposited on the downstream heating elements.

### Example 9

### Purity and Yield of Emitted Dose

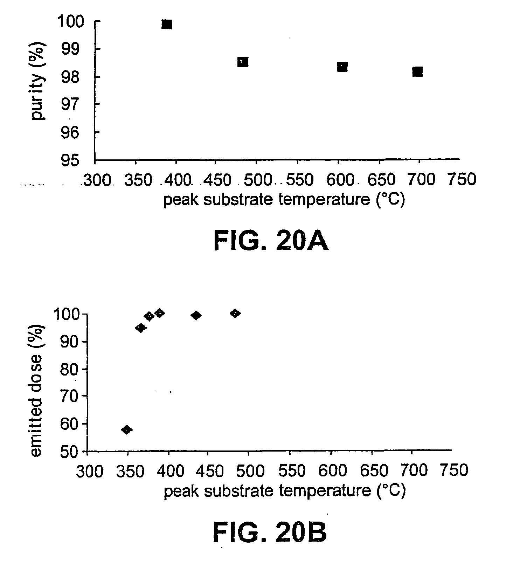

The purity and yield of emitted doses for devices as described in Example 1, except that the surface area of each support was 0.25 cm², are presented in **Figs. 20A** and **20B. Fig. 20A** shows that the purity of a 2.4 µm thick, 60 µg dose of fentanyl emitted from the device is greater than 98% when the peak temperature of the heating element is at least 375 °C. As shown in **Fig. 20B****,** at least 96% of the 2.4 µm thick, 60 µg dose of fentanyl disposed on a heating element was emitted from the device when heated to a temperature of at least 375 °C. For **Figs. 20A** and **20B****,** the condensation aerosols comprising fentanyl were characterized by a MMAD of 2.0 µm and a GSD of 1.8 µm.

## Claims

1. A device for entraining a substance within an airflow comprising:
a plurality of supports (40) disposed within an airway;
the airway (30, 31) comprising an inlet (35), and an outlet (37) and comprising a structure having a plurality of holes in fluid connection with the airway through which at least a portion of the airflow from the inlet to the outlet is directed to the plurality of supports;
a substance disposed on more than one of the plurality of supports; and
a mechanism configured to vaporize the substance from each of the plurality of supports upon the application of mechanical, acoustic, radiation, radio frequency, optical and/or thermal energy,
wherein the airway is configured to route an airflow (36) within the airway to entrain the substance in the airflow when released from a support to form a condensation aerosol in the airflow, and thereby reduce deposition of the substance (41) on surfaces in the downstream airflow.

2. The device of claim 1, further comprising an air bypass hole coupled to the outlet.

3. The device of claim 2, wherein the structure divides the airway into a first and second portion.

4. The device of claim 1, wherein the substance comprises at least one physiologically active compound.

5. The device of claim 1, wherein the substance comprises a pharmaceutical composition comprising at least one pharmaceutically acceptable excipient, and a therapeutically effective amount of at least one physiologically active compound.

6. The device of claim 4, wherein the compound is chosen from alprazolam, buprenorphine, clonindine, fentanyl, midazolam, pramipexole, ropinirole, and triazolam.

7. The device of claim 1, wherein at least one of the supports is an electrically resistive heating element.

8. The device of claim 7, wherein the electrically resistive heating element comprises a metal foil.

9. The device of claim 8, wherein the metal foil is arched.

10. The device of claim 1, wherein the plurality of supports comprises a plurality of electrically resistive heating elements.

11. The device of claim 1, wherein the substance is disposed on at least one of the supports as a layer.

12. The device of claim 11, wherein the thickness of the layer is between 0.01 µm and 10 µm.

13. The device of claim 10, wherein the same substance is disposed on each electrically resistive heating element.

14. The device of claim 10, wherein a different substance is disposed on at least two of the electrically resistive heating elements.

15. The device of claim 7, wherein the substance is coated as a layer on both sides of the electrically resistive heating element.

16. The device of claim 7, wherein the substance is coated as a layer on a single side of the electrically resistive heating element.

17. The device of claim 1, wherein the airway has a cross-sectional area between 0.5 cm² and 3 cm².

18. The device of claim 1, further comprising a flow control valve for controlling airflow rate through the airway.

19. The device of claim 1, further comprising
a dispensing unit comprising:
a first housing comprising a receptacle for a separable cartridge;
a controller for controlling release of the substance; and
a power source; and
a separable cartridge comprising:
a second housing;
the airway;
a mouthpiece coupled to the outlet;
the plurality of supports;
the substance; and
an actuation mechanism configured to transfer energy from the power source to the at least one of the plurality of supports.

20. The device of claim 20, further comprising an air bypass hole coupled to the outlet of the second housing.

21. A method of entraining a substance within an airflow comprising:
providing a plurality of supports (40) disposed within an airway, wherein the substance is disposed on at least one support of the plurality of supports;
the airway (30, 31) comprising an inlet (35) and an outlet (37) and having a plurality of holes in fluid connection with the airway through which at least a portion of the airflow from the inlet to the outlet is directed to the plurality of supports;
vaporizing the substance from at least one of the supports into the airflow by applying mechanical, acoustic, radiation, radio frequency, optical and/or thermal energy; and
routing an airflow (36) within the airway to entrain the substance in the airflow when released from the support to form a condensation aerosol in the airflow and to reduce deposits of the substance (41) on surfaces of supports in the downstream airflow.

22. The method of claim 21 wherein at least a portion of the airflow passes through a plurality of holes and is directed toward the support on which the substance is disposed.

23. The device according to claim 1 comprising an electrically resistive heating element **characterized by** comprising a metal foil having a thickness less than 0.00254 cm (0.001 inches) for vaporizing a substance disposed thereon to produce a condensation aerosol comprising the substance.

24. The device of claim 23, wherein the metal foil is stainless steel.

25. The device of claim 23, wherein the thickness of the metal foil is less than 0.00127 cm (0.0005 inches).

26. The device of claim 23, wherein the surface area of the metal foil is between 0.01 cm² and 50 cm².

27. The device of claim 23, wherein the metal foil comprises a metal layer plated on the metal foil.

28. The device of claim 27, wherein the thickness of the metal layer is between 0.001 µm and 3 µm.

29. The device of claim 23 or 27, wherein the metal foil is arched.

30. The device of claim 29, wherein the height of the arch is between 0.5 mm and 2 mm.

31. The device of claim 23, wherein the ratio of the surface area of the heating element, to the thermal mass of the heating element is greater than 100 cm²/J/°C.

32. The device of claim 23, wherein the ratio of the surface area of the heating element, to the thermal mass of the heating element is greater than 500 cm²/J/°C.

33. The device of claim 23, wherein the heating element can reach a temperature of at least 250°C in less than 250 msec.

34. The device of claim 23, wherein the heating element can reach a temperature of at least 250°C in less than 100 msec.

## Patentansprüche

1. Vorrichtung zum Zumischen einer Substanz in einen Luftstrom, umfassend:
eine Vielzahl an Trägerelementen (40), die in einem Luftschacht angeordnet sind;
wobei der Luftschacht (30, 31) einen Einlass (35) und einen Auslass (37) umfasst und eine Struktur mit einer Vielzahl an Löchern in Fluidverbindung mit dem Luftschacht umfasst, über die zumindest ein Teil des Luftstroms vom Einlass zum Auslass zur Vielzahl an Trägerelementen geleitet wird;
eine Substanz, die auf mehr als einem der Vielzahl an Trägerelementen angeordnet ist; und
einen Mechanismus, der konfiguriert ist, um die Substanz von jedem der Vielzahl an Trägerelementen bei der Beaufschlagung von mechanischer, akustischer, Strahlungs-, Hochfrequenz-, optischer und/oder Wärmeenergie verdampfen zu lassen,
worin der Luftschacht konfiguriert ist, um einen Luftstrom (36) innerhalb des Luftschachts so zu führen, dass die Substanz dem Luftstrom zugemischt wird, wenn sie von einem Trägerelement freigegeben wird, um ein Kondensationsaerosol im Luftstrom zu bilden, und dadurch ein Ablagern der Substanz (41) auf Oberflächen im Stromabwärtsluftstrom zu verringern.

2. Vorrichtung nach Anspruch 1, ferner umfassend ein Luftnebenstromloch, das mit dem Auslass verbunden ist.

3. Vorrichtung nach Anspruch 2, worin die Struktur den Luftschacht in einen ersten und einen zweiten Abschnitt unterteilt.

4. Vorrichtung nach Anspruch 1, worin die Substanz zumindest eine physiologisch aktive Verbindung umfasst.

5. Vorrichtung nach Anspruch 1, worin die Substanz eine pharmazeutische Zusammensetzung umfasst, die zumindest einen pharmazeutisch akzeptablen Hilfsstoff und eine therapeutisch wirksame Menge von zumindest einer physiologisch aktiven Verbindung umfasst.

6. Vorrichtung nach Anspruch 4, worin die Verbindung ausgewählt ist aus Alprazolam, Buprenorphin, Clonindin, Fenantyl, Midazolam, Pramipexol, Ropinirol und Triazolam.

7. Vorrichtung nach Anspruch 1, worin zumindest eines der Trägerelemente ein elektrisches Widerstands-Heizelement ist.

8. Vorrichtung nach Anspruch 7, worin das elektrische Widerstands-Heizelement eine Metallfolie umfasst.

9. Vorrichtung nach Anspruch 8, worin die Metallfolie gebogen ist.

10. Vorrichtung nach Anspruch 1, worin die Vielzahl an Trägerelementen eine Vielzahl von elektrischen Widerstands-Heizelementen umfasst.

11. Vorrichtung nach Anspruch 1, worin die Substanz auf zumindest einem der Trägerelemente als eine Schicht angeordnet ist.

12. Vorrichtung nach Anspruch 11, worin die Dicke der Schicht zwischen 0,01 µm und 10 µm beträgt.

13. Vorrichtung nach Anspruch 10, worin die Substanz auf jedem elektrischen Widerstands-Heizelement angeordnet ist.

14. Vorrichtung nach Anspruch 10, worin auf zumindest zwei der elektrischen Widerstands-Heizelemente eine unterschiedliche Substanz angeordnet ist.

15. Vorrichtung nach Anspruch 7, worin die Substanz als eine Schicht auf beiden Seiten des elektrischen Widerstands-Heizelements beschichtet ist.

16. Vorrichtung nach Anspruch 7, worin die Substanz als eine Schicht auf einer einzigen Seite des elektrischen Widerstands-Heizelements beschichtet ist.

17. Vorrichtung nach Anspruch 1, worin der Luftschacht eine Querschnittsfläche zwischen 0,5 cm² und 3 cm² aufweist.

18. Vorrichtung nach Anspruch 1, ferner umfassend ein Durchflussregelventil zum Steuern der Luftstromrate durch den Luftschacht.

19. Vorrichtung nach Anspruch 1, ferner umfassend
eine Spendereinheit, umfassend:
ein erstes Gehäuse, das eine Aufnahme für eine lösbare Kartusche umfasst;
ein Steuerelement zum Steuern der Freisetzung der Substanz; und
eine Spannungsquelle; und
eine lösbare Kartusche, umfassend:
ein zweites Gehäuse;
den Luftschacht;
ein mit dem Auslass verbundenes Mundstück;
die Vielzahl an Trägerelementen;
die Substanz; und
einen Betätigungsmechanismus, der konfiguriert ist, um Energie von der Spannungsquelle zu dem zumindest einen aus der Vielzahl an Trägerelementen zu übertragen.

20. Vorrichtung nach Anspruch 19, ferner umfassend ein Luftnebenstromloch, das mit dem Auslass des zweiten Gehäuses verbunden ist.

21. Verfahren zum Zumischen einer Substanz in einen Luftstrom, umfassend:
Bereitstellen einer Vielzahl an Trägerelementen (40), die in einem Luftschacht angeordnet sind, worin die Substanz auf zumindest einem Trägerelement der Vielzahl von Trägerelementen angeordnet ist;
den Luftschacht (30, 31), der einen Einlass (35) und einen Auslass (37) umfasst und eine Vielzahl an Löchern in Fluidverbindung mit dem Luftschacht aufweist, über die zumindest ein Abschnitt des Luftstroms vom Einlass zum Auslass zur Vielzahl an Trägerelementen geleitet wird;
Verdampfen der Substanz von zumindest einem der Trägerelemente in den Luftstrom unter Beaufschlagung von mechanischer, akustischer, Strahlungs-, Hochfrequenz-, optischer und/oder Wärmeenergie,
Führen eines Luftstroms (36) innerhalb des Luftschachts, sodass die Substanz dem Luftstrom zugemischt wird, wenn sie vom Trägerelement freigegeben wird, um ein Kondensationsaerosol im Luftstrom zu bilden und ein Ablagern der Substanz (41) auf Oberflächen von Trägerelementen des Stromabwärtsluftstroms zu verringern.

22. Verfahren nach Anspruch 21, worin zumindest ein Teil des Luftstroms durch eine Vielzahl an Löchern hindurchtritt und hin zum Trägerelement geleitet wird, auf dem die Substanz angeordnet ist.

23. Vorrichtung nach Anspruch 1, umfassend ein elektrisches Widerstands-Heizelement, **dadurch gekennzeichnet, dass** es eine Metallfolie mit einer Dicke von weniger als 0,00254 cm (0,001 Zoll) umfasst, um eine darauf angeordnete Substanz verdampfen zu lassen, um ein die Substanz umfassendes Kondensationsaerosol herzustellen.

24. Vorrichtung nach Anspruch 23, worin die Metallfolie rostfreier Stahl ist.

25. Vorrichtung nach Anspruch 23, worin die Dicke der Metallfolie weniger als 0,00127 cm (0,0005 Zoll) beträgt.

26. Vorrichtung nach Anspruch 23, worin die Oberfläche der Metallfolie zwischen 0,01 cm² und 50 cm² beträgt.

27. Vorrichtung nach Anspruch 23, worin die Metallfolie eine Metallschicht umfasst, die auf die Metallfolie aufgebracht ist.

28. Vorrichtung nach Anspruch 27, worin die Dicke der Metallschicht zwischen 0,001 µm und 3 µm beträgt.

29. Vorrichtung nach Anspruch 23 oder 27, worin die Metallfolie gebogen ist.

30. Vorrichtung nach Anspruch 29, worin der Bogen eine Höhe zwischen 0,5 mm und 2 mm beträgt.

31. Vorrichtung nach Anspruch 23, worin das Verhältnis der Oberfläche des Heizelements zur thermisch wirksamen Masse des Heizelements größer als 100 cm²/J/°C ist.

32. Vorrichtung nach Anspruch 23, worin das Vehrältnis der Oberfläche des Heizelements zur thermisch wirksamen Masse des Heizelements größer als 500 cm²/J/°C ist.

33. Vorrichtung nach Anspruch 23, worin das Heizelement in weniger als 250 ms eine Temperatur von zumindest 250 °C erreichen kann.

34. Vorrichtung nach Anspruch 23, worin das Heizelement in weniger als 100 ms eine Temperatur von zumindest 250 °C erreichen kann.

## Revendications

1. Dispositif pour entraîner une substance dans un écoulement d'air, comprenant :
une pluralité de supports (40) disposés dans une voie d'air ;
la voie d'air (30, 31) comprenant une entrée (35) et une sortie (37), et comprenant une structure ayant une pluralité de trous en connexion fluidique avec la voie d'air à travers lesquels au moins une portion d'écoulement d'air de l'entrée à la sortie est dirigée vers la pluralité de supports ;
une substance disposée sur plus d'un de la pluralité de supports ; et
un mécanisme configuré pour vaporiser la substance de chacun de la pluralité de supports lors de l'application d'une énergie mécanique, acoustique, de rayonnement, de radiofréquence, optique et/ou thermique,
où la voie d'air est configurée pour diriger un écoulement d'air (36) dans la voie d'air pour entraîner la substance dans l'écoulement d'air lorsqu'elle est libérée d'un support pour former un aérosol à condensation dans l'écoulement d'air, et pour réduire ainsi le dépôt de la substance (41) sur des surfaces dans l'écoulement d'air en aval.

2. Dispositif selon la revendication 1, comprenant en outre un trou de contournement d'air couplé à la sortie.

3. Dispositif selon la revendication 2, où la structure divise la voie d'air en une première et une seconde portion.

4. Dispositif selon la revendication 1, où la substance comprend au moins un composé physiologiquement actif.

5. Dispositif selon la revendication 1, où la substance comprend une composition pharmaceutique comprenant au moins un excipient acceptable du point de vue pharmaceutique, et une quantité efficace du point de vue thérapeutique d'au moins un composé physiologiquement actif.

6. Dispositif selon la revendication 4, où le composé est sélectionné parmi alprazolam, buprenorphine, clonindine, fentanyl, midazolam, pramipexole, ropinirole et triazolam.

7. Dispositif selon la revendication 1, où au moins un des supports est un élément de chauffage électriquement résistif.

8. Dispositif selon la revendication 7, où l'élément de chauffage électriquement résistif comprend une feuille métallique.

9. Dispositif selon la revendication 8, où la feuille métallique est arquée.

10. Dispositif selon la revendication 1, où la pluralité de supports comprend une pluralité d'éléments de chauffage électriquement résistifs.

11. Dispositif selon la revendication 1, où la substance est disposée sur au moins un des supports comme une couche.

12. Dispositif selon la revendication 11, où l'épaisseur de la couche est entre 0,01 µm et 10 µm.

13. Dispositif selon la revendication 10, où la même substance est disposée sur chaque élément de chauffage électriquement résistif.

14. Dispositif selon la revendication 10, où une substance différente est disposée sur au moins deux des éléments de chauffage électriquement résistifs.

15. Dispositif selon la revendication 7, où la substance est appliquée comme une couche sur les deux côtés de l'élément de chauffage électriquement résistif.

16. Dispositif selon la revendication 7, où la substance est appliquée comme une couche sur un seul côté de l'élément de chauffage électriquement résistif.

17. Dispositif selon la revendication 1, où la voie d'air a une zone en section transversale entre 0,5 cm² et 3 cm².

18. Dispositif selon la revendication 1, comprenant en outre une vanne de commande d'écoulement pour commander le débit d'écoulement d'air à travers la voie d'air.

19. Dispositif selon la revendication 1, comprenant en outre
une unité de distribution comprenant :
un premier boîtier comprenant un réceptacle pour une cartouche séparable ;
un dispositif de commande pour commander la libération de la substance ; et
une source de puissance ; et
une cartouche séparable comprenant :
un deuxième boîtier ;
la voie d'air ;
un embout couplé à la sortie ;
la pluralité de supports ;
la substance ; et
un mécanisme d'actionnement configuré pour transférer l'énergie de la source de puissance à au moins un, précité, de la pluralité de supports.

20. Dispositif selon la revendication 19, comprenant en outre un trou de contournement d'air couplé à la sortie du deuxième boîtier.

21. Procédé d'entraînement d'une substance dans un écoulement d'air, comprenant :
la réalisation d'une pluralité de supports (40) disposés dans une voie d'air, où la substance est disposée sur au moins un support de la pluralité de supports ;
la voie d'air (30, 31) comprenant une entrée (35) et une sortie (37) et ayant une pluralité de trous en connexion fluidique avec la voie d'air à travers lesquels au moins une portion de l'écoulement d'air de l'entrée à la sortie est dirigée vers la pluralité de supports ;
la vaporisation de la substance depuis au moins un des supports dans l'écoulement d'air par l'application d'une énergie mécanique, acoustique, de rayonnement, de radiofréquence, optique et/ou thermique, et
diriger un écoulement d'air (36) dans la voie d'air pour entraîner la substance dans l'écoulement d'air lorsqu'elle est libérée d'un support pour former un aérosol à condensation dans l'écoulement d'air, et pour réduire ainsi les dépôts de la substance (41) sur des surfaces de supports dans l'écoulement d'air en aval.

22. Procédé selon la revendication 21, où au moins une portion de l'écoulement d'air passe à travers une pluralité de trous et est dirigée vers le support sur lequel la substance est disposée.

23. Dispositif selon la revendication 1, comprenant un élément de chauffage électriquement résistif, caractérisé en comprenant une feuille métallique d'une épaisseur inférieure à 0,00254 cm (0,001 pouce) pour vaporiser une substance disposée sur celle-ci afin de produire un aérosol à condensation comprenant la substance.

24. Dispositif selon la revendication 23, où la feuille métallique est l'acier inoxydable.

25. Dispositif selon la revendication 23, où l'épaisseur de la feuille métallique est inférieure à 0,00127 cm (0,0005 pouce).

26. Dispositif selon la revendication 23, où l'aire de surface de la feuille métallique est entre 0,01 cm² et 50 cm².

27. Dispositif selon la revendication 23, où la feuille métallique comprend une couche métallique plaquée sur la feuille métallique.

28. Dispositif selon la revendication 27, où l'épaisseur de la couche métallique est entre 0,001 µm et 3 µm.

29. Dispositif selon la revendication 23 ou 27, où la feuille métallique est arquée.

30. Dispositif selon la revendication 29, où la hauteur de l'arc est entre 0,5 mm et 2 mm.

31. Dispositif selon la revendication 23, où le rapport de l'aire de surface de l'élément chauffant à la masse thermique de l'élément chauffant est supérieur à 100 cm²/J/°C.

32. Dispositif selon la revendication 23, où le rapport de l'aire de surface de l'élément chauffant à la masse thermique de l'élément chauffant est supérieur à 500 cm²/J/°C.

33. Dispositif selon la revendication 23, où l'élément chauffant peut atteindre une température d'au moins 250 °C en moins de 250 msec.

34. Dispositif selon la revendication 23, où l'élément chauffant peut atteindre une température d'au moins 250 °C en moins de 100 msec.
